(19) Europäisches Patentamt
European Patent Office
Office européen des brevets

(11) EP 1 657 235 B1

(12) EUROPEAN PATENT SPECIFICATION

(45) Date of publication and mention of the grant of the patent:
**21.09.2011 Bulletin 2011/38**

(21) Application number: **05077582.4**

(22) Date of filing: **10.11.2005**

(51) Int Cl.:
*C07C 215/60* (2006.01) *C07C 213/02* (2006.01)
*C07C 215/56* (2006.01) *C07K 16/00* (2006.01)
*G01N 33/48* (2006.01) *G01N 33/53* (2006.01)
*G01N 33/532* (2006.01)

(54) **Phenethanolamine-derived haptens, immunogens and conjugates comprising them and antibodies recognising said immunogenes and conjugates**

Phenethanolamine Abstammende Haptene, Immunogene und Konjugate die diese enthalten und Antikörper gegen solche immunogene und Konjugate

Haptènes derivés de la phenethanolamine, imunogènes et conjugués comprenant ces haptènes et anticorps reconnaissant lesdits immunogenes et conjugés

(84) Designated Contracting States:
**AT BE BG CH CY CZ DE DK EE ES FI FR GB GR HU IE IS IT LI LT LU LV MC NL PL PT RO SE SI SK TR**

(30) Priority: **10.11.2004 EP 04078100**

(43) Date of publication of application:
**17.05.2006 Bulletin 2006/20**

(73) Proprietor: **Randox Laboratories Ltd.**
**Crumlin, County Antrim BT29 4QY (GB)**

(72) Inventors:
- **McConnell, Robert Ivan**
  **Crumlin BT29 4QY**
  **Northern Ireland (GB)**
- **Fitzgerald, Stephen Peter**
  **Crumlin BT29 4QY**
  **Northern Ireland (GB)**
- **Benchikh, El Ouard**
  **Crumlin BT29 4QY**
  **Northern Ireland (GB)**
- **Lowry, Andrew Philip**
  **Crumlin BT29 4QY**
  **Northern Ireland (GB)**

(74) Representative: **O'Connell, Maura et al**
**FRKelly**
**4 Mount Charles**
**Belfast, Northern Ireland**
**BT7 1NZ (GB)**

(56) References cited:
**US-B1- 6 274 334**

- **SHELVER WEILIN L ET AL: "Production and characterization of a monoclonal antibody against the beta-adrenergic agonist ractopamine" JOURNAL OF AGRICULTURAL AND FOOD CHEMISTRY, vol. 48, no. 9, September 2000 (2000-09), pages 4020-4026, XP002318675 ISSN: 0021-8561**
- **SHELVER W L ET AL: "Development of an immunoassay for the [beta]-adrenergic agonist ractopamine" JOURNAL OF IMMUNOASSAY 2000 UNITED STATES, vol. 21, no. 1, 2000, pages 1-23, XP008043346 ISSN: 0197-1522**
- **REN, JIN-ZHI ET AL: "Synthesis of ritodrine" ZHONGGUO YIYAO GONGYE ZAZHI , 31(6), 241-242 CODEN: ZYGZEA; ISSN: 1001-8255, 2000, XP002372518**
- **HAASNOOT W ET AL: "IMMUNOFILTRATION AS SAMPLE CLEANUP FOR THE IMMUNOCHEMICAL DETECTION OF BETA-AGONISTS IN URINE" ANALYST, ROYAL SOCIETY OF CHEMISTRY, LONDON, GB, vol. 127, no. 1, 2002, pages 87-92, XP008043236 ISSN: 0003-2654**
- **SHELVER WEILIN L ET AL: "Application of a monoclonal antibody-based enzyme-linked immunosorbent assay for the determination of ractopamine in incurred samples from food animals" JOURNAL OF AGRICULTURAL AND FOOD CHEMISTRY, vol. 50, no. 10, 8 May 2002 (2002-05-08), pages 2742-2747, XP002372519 ISSN: 0021-8561**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **SHELVER WEILIN L ET AL: "Determination of ractopamine in cattle and sheep urine samples using an optical biosensor analysis: Comparative study with HPLC and ELISA." JOURNAL OF AGRICULTURAL AND FOOD CHEMISTRY, vol. 51, no. 13, 18 June 2003 (2003-06-18), pages 3715-3721, XP002372520 ISSN: 0021-8561**
- **TERANDO N H: "SYNTHESIS OF CARBON-14 RADIOLABELED RACTOPAMINE HYDROCHLORIDE" JOURNAL OF LABELLED COMPOUNDS AND RADIOPHARMACEUTICALS, vol. 31, no. 9, 1992, pages 651-658, XP009063523 ISSN: 0362-4803**
- **MADDING, G. D.: "Synthesis of tritium-labeled isoxsuprine hydrochloride" JOURNAL OF LABELLED COMPOUNDS , 7(4), 393-7 CODEN: JLCAAI; ISSN: 0022-2135, 1971, XP009063548**
- **MILES G SIEGEL ET AL: "The use of high-throughput synthesis and purification in the preparation of a directed library of adrenergic agents" MOLECULAR DIVERSITY, ESCOM SCIENCE PUBLISHERS, LEIDEN, NL, vol. 3, no. 2, 1998, pages 113-116, XP002079084 ISSN: 1381-1991**
- **BRUNSWICK D J ET AL: "Radioimmunoassay of imipramine and desmethylimipramine", LIFE SCIENCES, PERGAMON PRESS, OXFORD, GB, vol. 22, no. 2, 1 January 1978 (1978-01-01), pages 137-146, XP023739312, ISSN: 0024-3205, DOI: DOI:10.1016/0024-3205(78)90530-1 [retrieved on 1978-01-01]**


Remarks:

The file contains technical information submitted after the application was filed and not included in this specification

## Description

The present invention relates to a method for preparing haptens that are useful for the preparation of immunogens, antibodies and conjugates, for use in competitive immunoassays for the detection of phenethanolamines such as, but not limited to, ractopamine, isoxsuprine and ritodrine.

The present disclosure also relates to a method and kit for detecting or determining phenethanolamines such as, but not limited to, ractopamine, isoxsuprine and ritodrine.

By "detecting" is meant qualitatively analysing for the presence or absence of a substance. By "determining" is meant quantitatively analysing for the amount of a substance.

Ractopamine is a phenethanolamine leanness-enhancing agent recently approved as a feed additive for swine in the United States. Hogs administered dietary ractopamine show increased growth rates and feed efficiencies and decreased fat deposition, relative to untreated animals.

Phenethanolamine β-agonists have a history of being used for off-label purposes by livestock producers hoping to improve the economics of livestock production. Improper use of β-agonists can cause a serious risk to human health due to the residues they leave in the meat and other foodstuffs of animal origin. Indeed, in Europe, all β-agonists are banned for use in livestock and for improving athletic performance under EU Directive 96/22/EC.

The presence of drug residues in animal tissues is a concern for food safety, especially when the compound has been used illegally or in a manner proscribed by regulatory officials (off-label use). In an effort to combat such illicit use of β-agonist compounds, regulatory organisations around the world test animal tissues and body fluids for the presence of such illicit drugs.

Specific binding actions, such as antibody-antigen interactions, have been used extensively in immunoassays to detect a variety of substances present in biological fluids. Thus, for example, radioimmunoassays could be used for the determination of phenethanolamines such as ractopamine, isoxsuprine and ritodrine. Radioimmunoassays are very sensitive but do require radionucleotide tracers. Enzyme-linked immunosorbent assays (ELISAs) are a non-radioactive alternative which could be used for the qualitative and quantitative determination of phenethanolamines such as, but not limited to, ractopamine, isoxsuprine and ritodrine.

Haasnoot et al (The Analyst (1994), 119, "Determination of fenoterol and ractopamine in urine by enzyme immunoassay", 2675-2680) disclose a urinary enzyme immunoassay for ractopamine. The Haasnoot antibody was derived from a fenoterol derivative and showed 20% cross-reactivity with ractopamine, but the analysis of ractopamine did not correlate well with GC-MS analysis. Elliott et al (The Analyst (1998), 123, "Screening and confirmatory determination of ractopamine residues in calves treated with growth promoting doses of the β-agonist", 1103-1107) disclose an immunoassay for ractopamine residues. The Elliott antibody, when used in an immunoassay where the samples were enzymatically hydrolysed prior to analysis, correlated well with LC-MS-MS but, as with Haasnoot *et al,* the antibodies were raised in a non-targeted fashion, against a fentoterol derivative.

Shelver and Smith (Journal of Immunoassay (2000), 21(1), "Development of an immunoassay for the β-adrenergic agonist ractopamine", 1-23) disclose preparation of polyclonal antibodies generated from ractopamine-hemiglutarate-KLH. The hapten is prepared by reaction of ractopamine with glutaric anhydride, followed by conjugation with an antigenicity-conferring carrier material. The method employed to synthesize ractopamine-hemiglutarate is uncontrolled and will result in derivatisation of ractopamine at any one of positions 1, 10, 10' and N. The Shelver and Smith antibody generated shows a sensitivity ($IC_{50}$) of 4.2ng/ml toward ractopamine. The antibody also exhibits 33% cross-reactivity with dobutamine.

Shelver et al (J. Agric. Food Chem. 48 (2000), "Production and characterisation of a monoclonal antibody against the β-adrenergic agonist ractopamine", 4020-4026 and their US Patent No. 6,274,334) disclose the generation of a monoclonal antibody, in which ractopamine-glutarate-keyhole limpet haemocyanin was used as the antigen for antibody generation. The antibody clone selected (5G10) shows 5.3% cross-reactivity with dobutamine and 3.6% cross-reactivity with ritodrine.

A polyclonal antibody is generated by repeated immunisation of a non-human mammalian host with the target immunogen. The resulting antiserum is harvested and the antibodies isolated. To generate a monoclonal antibody, lymphocytes from an immunized animal are fused with a myeloma cell line to produce hybrid cells or hybridomas. These hybridomas can be cloned for production of the secreted monoclonal antibody. A monoclonal antibody will recognize a single epitope, whereas a polyclonal antiserum may recognize several epitopes on the same antigen. Hence, monoclonal antibody technology can usually be applied to generate an antibody with greater specificity for the target immunogen.

In the aforementioned Shelver *et al,* a 'scattergun' approach has been used to generate polyclonal antibodies to ractopamine. The uncontrolled method used to prepare the ractopamine hapten suggests that the resulting immunogen is composed of a mixture of ractopamine linked to keyhole limpet haemocyanin (KLH) at any one of positions 1, 10, 10' and N. Immunisation with such an immunogen will result in the generation of antisera with a diversity of specificity. This is borne out in the cross-reactivity data presented in the Shelver reference. Their polyclonal antibody cross-reacts 33% with dobutamine, while theirmonoclonal antibody cross-reacts 5.3% with dobutamine. In contrast, the targetted introduction of a crosslinker group onto a single phenolic hydroxyl group of the target hapten, according to the present invention, produces a surprising effect (a heterofunctional crosslinker as defined herein, is a structure incorporating one or more functional groups containing one or more hetero-atoms, that links, for example through covalent bonding, with a substrate (in this case a hapten), the crosslinker also being able to link to a peptide, polypeptide or protein or a detectable labelling agent). Antibodies generated to the present haptens are highly specific and do not cross-react significantly with dobutamine. In the case of ractopamine, the antibody generated to the position 10 derivative cross-reacts 0.186% with dobutamine, while the antibody generated to the position 10' derivative exhibits cross-reactivity of <0.8% with dobutamine. The only explanation for the cross-reactivity of the Shelver antibodies with dobutamine is that Shelver *et al* have generated antibodies to ractopamine derivatised at the position 1 hydroxyl (hydroxyl group on the aliphatic chain). This hydroxyl group is absent in dobutamine. The presence of a single hydroxyl group on one of the aromatic rings of the hapten derivatives used in the present application results in a high degree of specificity for ractopamine, isoxsuprine and ritodrine. The antibodies of the present application are also considerably more sensitive than the Shelver polyclonal and monoclonal antibodies. For ractopamine, the antibody to the position 10 derivative has an $IC_{50}$ of 0.082ng/ml, while the antibody to the position 10' derivative has an $IC_{50}$ of 0.202ng/ml. The present antibodies exhibit improved specificity and sensitivity. The present application illustrates how a superior antibody can be generated using polyclonal antibody technology and the process of targeted derivatisation.

None of the prior art known to the inventors either discloses or suggests preparing phenethanolamines haptens such as, but not limited to, ractopamine, isoxsuprine or ritodrine haptens by the method disclosed herein. The present method allows for the controlled coupling of a crosslinking group to a single phenolic hydroxyl group of the hapten. The present invention, by allowing the preparation of haptens and, therefore, immunogens derivatised at a single phenolic hydroxyl group, facilitates preparation of antibodies to immunogens derivatised at a single phenolic hydroxyl group. None of the prior art discloses or suggests targeted derivatisation of phenethanolamines such as, but not limited to, ractopamine, isoxsuprine and ritodrine analogues at a single phenolic hydroxy group. Such haptens also form part of the present invention.

## BRIEF DESCRIPTION OF DRAWINGS

**Figure 1** shows the structures of ractopamine, ritodrine and isoxsuprine.

**Figure 2** shows the structures of Haptens A, B, C, D and E.

**Figures 3-7** show reaction schemes for the preparation of Haptens / Immunogens A, B, C, D and E, respectively.

## DETAILED DESCRIPTION OF THE INVENTION

The present disclosure describes biaromatic haptens derivatised with crosslinkers at a single phenolic hydroxyl group. The structures of ractopamine, isoxsuprine and ritodrine are illustrated in Figure 1.

The disclosure also provides a hapten derivatised with a crosslinker at $Z_1$, in which the hapten has the structural formula I

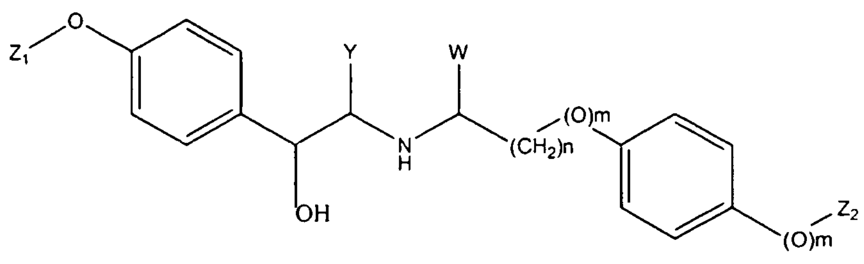

in which $Z_1$ is a crosslinker and $Z_2$ is H; Y is independently H or $CH_3$; W is independently H or $CH_3$; n is independently

1 or 2; and m is independently 0 or 1. Additionally, the invention provides a hapten derivatised with a crosslinker at $Z_2$, in which the hapten has the structural formula II

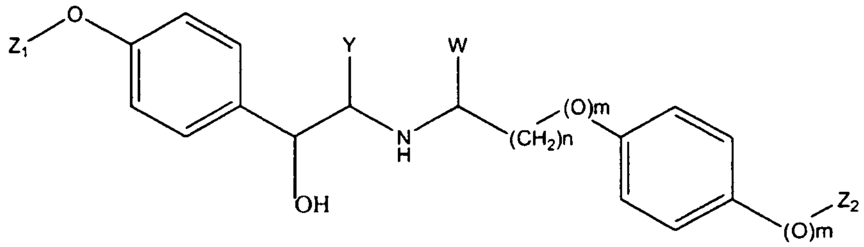

in which $Z_1$ is H and $Z_2$ is a crosslinker; Y is independently H or $CH_3$; W is independently H or $CH_3$; n is independently 1 or 2; and m is independently 0 or 1.

More preferably, the haptens have structural formulae A, B, C, D or E as illustrated in Figure 2.

In a further aspect, the invention provides a method of preparing a hapten of formula I

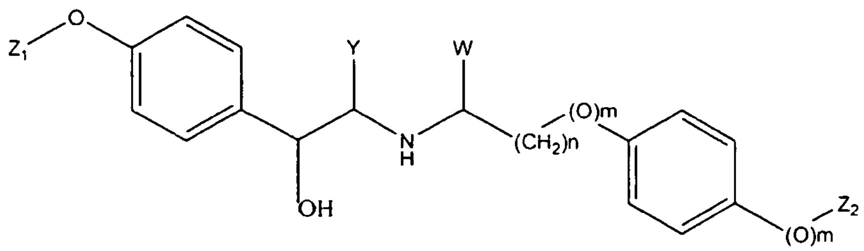

__Hapten I__

suitable for use in raising an antibody capable of binding with at least one structural epitope of a phenethanolamine such as, but not limited to, ractopamine, isoxsuprine or ritodrine, the method comprising the step of reacting a phenethanolamine derivative of the formula D, in which $Z_1$ is a crosslinker, with a phenylalkylcarbonyl derivative of the formula E, in which $Z_2$ is H; Y is independently H or $CH_3$; W is independently H or $CH_3$; n is independently 1 or 2; and m is independently 0 or 1

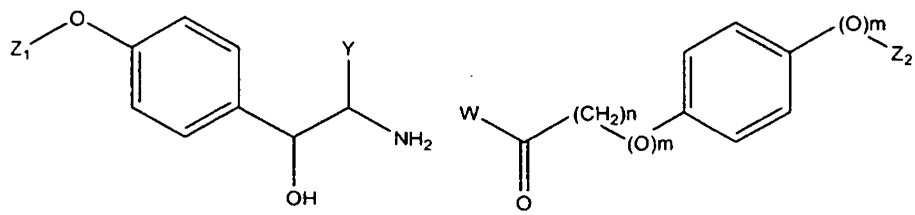

D E

Preferably, the phenethanolamine derivative of the formula D is prepared by reacting octopamine hydrochloride with $Z_1X_1$, in which $X_1$ is a halide, preferably a bromide. More preferably, the amine group of the octapamine hydrochloride is temporarily protected with BOC, by reacting the octapamine hydrochloride with di-tert-butyldicarbonate before reacting the BOC-protected octapamine hydrochloride with the $Z_1X_1$, the BOC group being subsequently removed.

In a further aspect, the invention provides a method of preparing a hapten of formula II

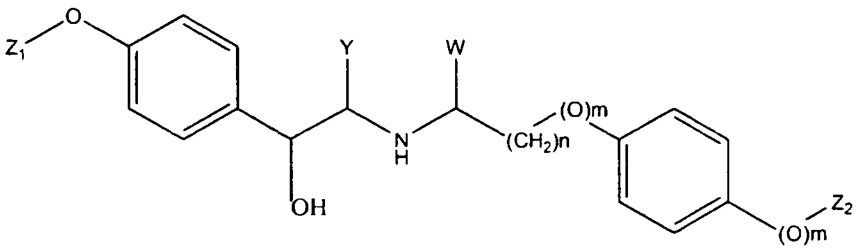

**Hapten II**

suitable for use in raising an antibody capable of binding with at least one structural epitope of a phenethanolamine such as, but not limited to, ractopamine, isoxsuprine or ritodrine, the method comprising the step of reacting a phenethanolamine derivative of the formula D, in which $Z_1$ is H, with a phenylalkylcarbonyl derivative of the formula E, in which $Z_2$ is a crosslinker; Y is independently H or $CH_3$; W is independently H or $CH_3$; n is independently 1 or 2; and m is independently 0 or 1

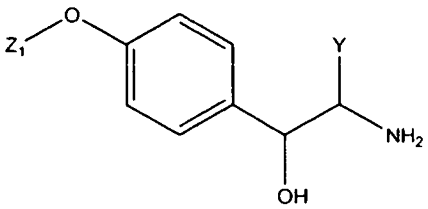

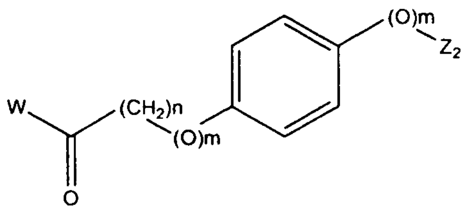

**D** **E**

Preferably, the phenylalkylcarbonyl derivative of the formula E is prepared by reacting 4-(4-hydroxyphenyl)-2-butanone with $Z_2X_1$, in which $X_1$ is a halide, preferably a bromide.

Preferably, the crosslinker is $-R-X_2$. Preferably, R is a bivalent link and $X_2$ is a heterofunctional group. By "heterofunctional" is meant a functional group containing at least one hetero-atom that is able to link the R of the crosslinker with either antigenicity-conferring carrier materials, to form immunogens or, alternatively, detectable labelling agents, to form conjugates. More preferably, the $X_2$ heterofunctional group is capable of reacting with either antigenicity-conferring carrier materials, to form immunogens or, alternatively, detectable labelling agents, to form conjugates.

Most preferably, R is selected from the group comprising a substituted or unsubstituted, straight or branched chain, saturated or unsaturated alkylene group and a substituted or unsubstituted arylene group such as a benzylene group and $X_2$ is selected from the group comprising a carboxylic acid or an ester thereof; an amine; a maleimide, a halocarboxylic acid or an ester thereof, an aldehyde, a dithiopyridyl group, a vinylsulphone group, and a thiocarboxylic acid or an ester thereof. More preferably, R is a $C_{1-5}$, most preferably a $C_{1-3}$, substituted or unsubstituted, straight chain, saturated alkylene group. Advantageously, $X_2$ is selected from a carboxylic acid or an ester thereof, a thiocarboxylic acid or an ester thereof, a dithiopyridyl, a maleimide, or an aldehyde group. Most advantageously, $X_2$ is a carboxyl group (COOH) or a thioacetyl ($SCOCH_3$) group.

The resulting haptens are deliberately derivatised at a single phenolic hydroxyl group of phenethanolamines such as, but not limited to, ractopamine, isoxsuprine and ritodrine.

The resulting haptens are employed in the preparation of immunogens by coupling them to modified or non-modified antigenicity-conferring carrier materials to provide immunogens for antibody production and conjugates (tracers) that have excellent sensitivity and specificity for the detection or determination of phenethanolamines such as, but not limited to, ractopamine, isoxsuprine and ritodrine.

The invention therefore provides a method of making an immunogen comprising a hapten prepared in accordance with the present invention, coupled to an antigenicity-conferring carrier material. Preferably, the carrier material is a protein, a protein fragment, a synthetic polypeptide or a semi-synthetic polypeptide.

The immunogens obtained are then administered to mammalian hosts to elicit production of specific antibodies, preferably polyclonal antibodies, which are then used to develop competitive immunoassays for phenethanolamines

such as, but not limited to, ractopamine, isoxsuprine and ritodrine, employing haptens conjugated to labelling agents as conjugates or detection reagents.

In a still further aspect, the present disclosure concerns antibodies raised against the immunogens of the present invention, the antibodies being capable of binding with at least one structural epitope of phenethanolamines such as, but not limited to, ractopamine, isoxsuprine and ritodrine. Preferably, the antibodies are fixed on a backing substrate. More preferably, the antibodies show a cross reactivity, when compared to 100% for ractopamine, isoxsuprine and ritodrine, of less than 5% for dobutamine. More preferably, the antibodies show a cross reactivity, when compared to 100% for ractopamine and isoxsuprine of less than 2.5%, more preferably less than 1.5%, for dobutamine. Separately or additionally, the antibodies raised to ractopamine show a cross reactivity, when compared to 100% for ractopamine, of less than 0.75%, preferably less than 0.6%, for ritodrine and isoxsuprine. Separately or additionally, the antibodies raised to isoxsuprine show a cross reactivity, when compared to 100% for isoxsuprine, of less than 0.75%, preferably less than 0.6%, more preferably less than 0.25%, for each of ritodrine and ractopamine. Separately or additionally, the antibodies raised to ritodrine show a cross reactivity, when compared to 100% for ritodrine, of less than 0.75%, preferably less than 0.6%, for ractopamine.

In a still further aspect, the present invention comprises a method of making a conjugate comprising a hapten prepared in accordance with the method of the present invention covalently bonded to a detectable labelling agent. Preferably, the labelling agent is selected from an enzyme, a luminescent substance, a radioactive substance, or a mixture thereof. More preferably, the labelling agent is an enzyme, preferably a peroxidase, most preferably horseradish peroxidase (HRP). Alternatively, or additionally, the luminescent substance may be a bioluminescent, chemiluminescent or fluorescent material.

The invention further provides a process of preparing the antibodies, the process comprising the steps of immunising a non-human animal, preferably a vertebrate non-human animal, most preferably a mammalian non-human animal, by repeated administration of an immunogen prepared in accordance with the present invention, and collecting the resulting serum from the immunised animal. Preferably, the process further comprises fixing said serum antibodies to a backing substrate, preferably a solid support, most preferably a polystyrene solid support. Preferably, the antibodies are polyclonal. Alternatively, the antibodies are monoclonal.

In a still further aspect, the present disclosure relates a method for detecting or determining phenethanolamines such as, but not limited to, ractopamine, isoxsuprine and ritodrine in a sample, the method comprising contacting the sample with at least one conjugate of the present invention, and with at least one antibody of the present invention; detecting or determining bound conjugate; and deducing from a calibration curve the presence of, or the amount of, the phenethanolamine in the sample.

In a further aspect, the disclosure includes a kit for detecting or determining phenethanolamines such as, but not limited to, ractopamine, isoxsuprine or ritodrine, the kit including at least one conjugate prepared in accordance with the present invention; and at least one antibody prepared in accordance with the present invention. The kit may optionally include instructions for the use of said conjugates and said antibodies for detecting or determining phenethanolamines such as, but not limited to, ractopamine, isoxsuprine or ritodrine in a sample.

The methods and kits using antibodies and conjugates raised to ractopamine of the present invention show an $IC_{50}$ of less than 2.5, preferably less than 1.5, more preferably less than 0.75, most preferably less than 0.25, ng/ml for ractopamine. The methods and kits, using antibodies and conjugates raised to isoxsuprine, show an $IC_{50}$ of less than 2.5, preferably less than 1.5, more preferably less than 0.75, more preferably less than 0.25, ng/ml for isoxsuprine. The methods and kits, using antibodies and conjugates raised to ritodrine, show an $IC_{50}$ of less than 250, preferably less than 150, ng/ml for ritodrine

Preferably, the antibodies are provided, fixed onto a backing substrate, the backing substrate preferably being a solid support which is, for example, a polystyrene solid support. This can take the form of a microtitre plate or, alternatively, a Biochip (Trade Mark) such as is disclosed in US-A-6,498,010. Phenethanolamines such as, but not limited to, ractopamine, isoxsuprine or ritodrine, if present in the standard and sample, compete with conjugates comprising haptens covalently bonded to a detectable labelling agent such as, but not restricted to, horseradish peroxidase, for the limited number of antibody sites on the backing substrate. After incubation at room temperature to allow a competition reaction to take place, the backing substrate is then washed to remove excess reagents. If the labelling agent is horseradish peroxidase, its substrate is then added and an incubation period follows to allow maximum signal development. In an ELISA format, the colour development is then stopped by the addition of acid and this produces a colour change from blue to yellow. The absorbance is read at 450nm. A standard curve is then constructed to determine the concentration of the phenethanolamine such as, but not limited to, ractopamine, isoxsuprine or ritodrine in the sample and the standard. Alternatively, in the Biochip format, the signal generated is typically chemiluminescent and the light level generated is quantified by a charge coupled device (CCD) camera to determine the concentration of the phenethanolamines such as, but not limited to, ractopamine, isoxsuprine and ritodrine in the sample or standard.

Preferably, the sample is a solution, such as a biological fluid. More preferably, the sample is serum or urine.

Urine samples may be analysed following a dilution method. However, if the urine appears dirty or a lower

detection limit is required, an appropriate extraction method should be used. Simple dilution involves centrifuging the urine sample at, for example, 13000rpm for 10 minutes and then diluting the centrifuged urine ten fold in diluted diluent/ wash buffer, following which the sample can be applied to the backing substrate.

Intra-assay precision of the ELISA kit was determined employing negative urine samples spiked with 1ng/ml and 5ng/ml. The results were 5.95 and 6.62% CV, respectively. Inter-assay precision of the ELISA kit for the same spiked urine samples was also determined and the results were 11.83 and 5.90% CV, respectively.

In the method and kit of the present disclosure, it is preferred that the respective crosslinkers (of the immunogen and the conjugate) are different.

In a further aspect, the present disclosure involves the use of the conjugates of the present invention, or a mixture thereof, with the antibodies of the present invention, or a mixture thereof, to detect or determine phenethanolamines such as, but not limited to, ractopamine, isoxsuprine or ritodrine in samples such as tissues or biological fluids.

## Preparation of Haptens

The hapten [10-O-(carboxypropyl)]ractopamine ether(Hapten **A -** derivatised at position 10) was prepared in three steps according to Figure **3** of the accompanying drawings, from 4-(4-hydroxyphenyl)-2-butanone, **2.** The reaction of 4-(4-hydroxyphenyl)-2-butanone, **2** with ethyl 4-bromobutyrate in dimethylformamide (DMF) in the presence of sodium hydride (NaH), gives ethyl 4-[4-(3-oxobutyl)phenoxy]butanoate **3.** The ketoester, **3,** obtained was reacted with octopamine hydrochloride, **4,** in methanol in the presence of triethylamine (TEA) and sodium cyanoborohydride (Na $BH_3CN$) to give 10-O-[3-(ethoxycarbonyl)propyl]ractopamine ether **5,** in moderate yields. The hapten **A** was obtained after saponification of the ester, **5,** with sodium hydroxide (NaOH) (2N) in methanol.

The hapten 10-O-(4-carboxybenzyl)ractopamine ether (hapten **B -** derivatised at position 10) was prepared by the same method as hapten **A** in three steps according to Figure **4** of the accompanying drawings, from 4-(4-hydroxyphenyl)-2-butanone, **2.** The reaction of **2** with methyl 4-(bromomethyl)benzoate, **6,** in DMF in the presence of NaH, gives methyl 4-[4-(3-oxobutyl)phenoxymethyl] benzoate, **7.** The condensation of the keto ester, **7** with octopamine hydrochloride, **4** in methanol in the presence of triethylamine (TEA) and sodium cyanoborohydride gives the ester **8.** The hapten **B** was obtained after saponification of the ester **8** with sodium hydroxide (2N) in methanol.

The hapten 10'-O-(3-carboxypropyl)ractopamine ether (Hapten **C** - derivatised at position 10') was prepared in six steps, according to Figure **5** of the accompanying drawings, from octopamine hydrochloride **4.** The N-BOC protected octopamine, **9** was obtained by reaction of **4** with di-tert-butyldicarbonate ($BOC_2O$) in methanol in the presence of TEA. The reaction of N-BOC octopamine, **9** with ethyl-4-bromobutyrate in acetonitrile in the presence of potassium carbonate ($K_2CO_3$) gives the ester **10** in good yield. The treatment of **10** with trifluoroacetic acid in dichloromethane gives ethyl 4-[4-(2-amino-1-hydroxyethyl)phenoxy] butanoate, **11.** The condensation of octopamine derivative **11** with 4-(4-hydroxyphenyl)-2-butanone **2** in methanol in the presence of TEA and sodium cyanoborohydride ($NaBH_3CN$) gives 10'-O-[3-(ethoxycarbonyl)propyl] ractopamine ether **12.** The hapten 10'-O-(3-carboxypropyl)ractopamine ether, Hapten **C**, was obtained after saponification of the ester **12** by using sodium hydroxide (2N) in methanol.

The hapten p-(carboxymethyl)isoxsuprine (Hapten D) was prepared in four steps according to Figure **6** of the accompanying drawings, from $\alpha$-(1-aminoethyl)-4-hydroxybenzyl alcohol hydrochloride **13.** The isoxsuprine **15** was obtained by reaction of **13** with phenoxy-1-propanone **14** in methanol in the presence of sodium cyanoborohydride and triethyl amine. The ester **16** was obtained by selective alkylation of isoxsuprine **15** with t-butyl bromoacetate in acetonitrile in the presence of potassium carbonate. The treatment of the ester **16** with trifluoroacetic acid in dichloromethane gave the p-(carboxymethyl)isoxsuprine, Hapten **D**.

The hapten p-(carboxymethyl)ritodrine (Hapten **E**) was prepared in four steps according to Figure **7** of the accompanying drawings, from 2-(4-hydroxyphenyl)ethanol **17.** The reaction of **17** with t-butyl bromoacetate in acetonitrile in the presence of potassium carbonate at 60°C gives 2-[4-(t-butoxycarbonylmethoxy)phenyl]ethanol **18.** The oxidation of the alcohol **18** with pyridinium chlorochromate (pcc) in dichloromethane gives the 2-[4-(t-butoxycarbonylmethoxy) phenyl]ethanal **19.** The ester **20,** obtained by condensation of the aldehyde **19** with $\alpha$-(1-aminoethyl)-4-hydroxybenzyl alcohol hydrochloride **13** in methanol in the presence of sodium cyanoborohydride and triethylamine. Deprotection of the t-butyl protecting group of the ester **20** with 4 M HCl in dioxane at room temperature gave Hapten **E** in moderate yield.

## Preparation of Immunogens and Conjugates

Although the haptens of the present invention provide defined structural epitopes, they are not in themselves immunogenic and therefore need to be conjugated to carrier materials, which will elicit an immunogenic response when administered to a host animal. Appropriate carrier materials commonly contain poly(amino acid) segments and include polypeptides, proteins and glycoproteins. Illustrative examples of useful carrier materials are bovine serum albumin (BSA), egg ovalbumin, bovine gamma globulin, thyroxine binding globulin, keyhole limpet haemocyanin (KLH) etc.

Alternatively, synthetic poly(amino acids) having a sufficient number of available amino groups, such as lysine, may be employed, as may other synthetic or natural polymeric materials bearing reactive functional groups. In particular, carbohydrates, yeasts or polysaccharides may be conjugated to the hapten to produce an immunogen.

Each hapten prepared in accordance with the present invention can also be coupled to a detectable labelling agent such as an enzyme (for example, horseradish peroxidase), a substance having luminescent, chemiluminescent or fluorescent properties or a radioactive label for the preparation of conjugates (or detection reagents) for use in the immunoassays. The fluorescent substance may be, for example, a monovalent residue of fluorescein or a derivative thereof.

In order to confirm that adequate conjugation of hapten to carrier material has been achieved, prior to immunisation, each immunogen is evaluated using matrix-assisted UV laser desorption /ionisation time-of-flight mass spectroscopy (MALDI-TOF MS). Each of the immunogens of the present invention is suitable for immunisation, in order to produce antibodies for the detection of phenethanolamines such as, but not limited to, ractopamine, isoxsuprine and ritodrine.

**General Procedure for MALDI-TOF Analysis of Immunogens.**

MALDI-TOF mass spectrometry was performed using a Voyager STR Biospectrometry Research Station laser-desorption mass spectrometer coupled with delayed extraction. An aliquot of each sample to be analysed was diluted in 0.1 % aqueous trifluoroacetic acid (TFA) to create 1mg/ml sample solutions. Aliquots (1 μl) were analysed using a matrix of Sinapinic acid and bovine serum albumin (Fluka) was used as an external calibrant.

**Preparation of Antisera**

In order to generate polyclonal antisera, each immunogen of the present invention is mixed with Freund's Adjuvant and the mixture is injected into a host animal, such as rabbit, sheep, mouse, guinea pig or horse. Further injections (boosts) are made and serum is sampled for evaluation of the antibody titre. When the optimal titre has been attained, the host animal is bled to yield a suitable volume of specific antiserum. The degree of antibody purification required depends on the intended application. For many purposes, there is no requirement for purification, however, in other cases, such as where the antibody is to be immobilised on a solid support, purification steps can be taken to remove undesired material and eliminate non-specific binding.

The specific antibodies prepared in this invention are useful as reagents in immunoassays for the detection or determination of phenethanolamines such as, but not limited to, ractopamine, isoxsuprine and ritodrine in biological fluids.

**EXAMPLES**

**Example 1: Preparation of Ethyl 4-[4-(3-oxobutyl)phcnoxy]butanoate 3**

To a suspension of sodium hydride (60% dispersion in mineral oil) (7.3g, 0.183 mole) in anhydrous DMF (100ml) under nitrogen atmosphere was added drop-wise 4-(4-hydroxyphenyl)-2-butanone **2** (20.0g, 0.122 mole) in anhydrous DMF (100ml) and the mixture was then stirred at room temperature for one hour. To this mixture was added drop-wise ethyl 4-bromobutyrate (28.5g, 0.146 mole) in anhydrous DMF (50ml), and the mixture was then stirred at 60°C overnight. The DMF was removed under reduced pressure and water (200ml) was then added to the residue and extracted with ethyl acetate (3 x 200ml). The combined organic phases were washed with brine (1 x 200ml), dried over magnesium sulfate, filtered and evaporated to dryness. The residue obtained was purified by flash chromatography on silica gel using 20% ethyl acetate in hexane as eluant to give **3** as a colourless oil (16.95g, 50%) .
I.R (Film): 1732; 1720; 1612.5; 1512.9; 1245.1 and 1178.

**Example 2: Preparation of 10-O-[3-(Ethoxycarbonyl)propyl]ractopamine ether 5**

To a solution of **3** (16.95g, 0.061 mole) and octopamine HCl (11.57g, 0.061 mole) in methanol (300ml) was added TEA (12.35g, 0.122 mole) and sodium cyanoborohydride (3.83g, 0.061 mole). The mixture was stirred at 50°C overnight. Solvents were removed *in vacuo.* To the residue were added HCl (1N) (150ml) and water (150ml) and washed with ether (1 x 300ml). The aqueous phase was adjusted to pH12-13 using sodium hydroxide (6N) and extracted with ethyl acetate (3 x 250ml). The organic layers were combined, dried over magnesium sulfate, filtered and evaporated to dryness. The crude product was purified by flash chromatography on silica gel using 5-10% methanol in chloroform to give the title compound **5** (7g, 28%) as a white solid. I.R (KBr): 3506;3301.2; 1735.8; 1613.2; 1512.6; 1374.03; 1244.1; 1176.9 and 1046.5.

**Example 3: Preparation of 10-O-(3-Carboxypropyl)ractopamine ether (Hapten A).**

To a solution of the ester 5 (7g, 0.169 mole) in methanol (90ml) was added sodium hydroxide (2N) (30ml). The mixture was stirred at room temperature for 16 hours. The solvents were removed *in vacuo* and water (50ml) was added. The pH of the solution was adjusted to 3-4 by addition of HCl (1N) and the resultant precipitate obtained was collected by filtration and dried in a desiccator over $P_2O_5$. The hapten **A** was obtained as a white solid (5.64g, 86%).

**Example 4: Preparation of Methyl 4-[4-(3-oxobutyl)phenoxymethyl]benzoate 7.**

The ester 7 was obtained by using the same method as described in Example 1 using 4-(4-hydroxyphenyl)-2-butanone, **2** (20g, 0.122 mole), sodium hydride (7.3g, 0.183 mole) and methyl 4-(bromomethyl) benzoate (33.44g, 0.146 mole) **6.** The title compound **7** was obtained as a clear oil (23.2g, 61 %).

**Example 5: Preparation of 10-O-[4- (Methoxycarbonyl)benzyl]ractopamine ether 8.**

The ester **8** was obtained by using the same method as described in Example **2** for the preparation of **5**, using the ester **7** (10.3g, 0.33 mole), octopamine hydrochloride **4** (6.26g, 0.33 mole), triethylamine (6.68g, 0.066 mole) and sodium cyanoborohydride (2.07g, 0.33 mole). The ester **8** was obtained in moderate yield (7g, 50%).
I.R (KBr): 3171.4; 3025.6; 2942.3; 2850.69; 1717.08; 1612.84; 1595.68; 1511.9; 1281.1; 1250.1 and 1107.

**Example 6: Preparation of 10-O-(4-Carboxybenzyl)ractopamine ether (hapten B).**

The hapten B was obtained in 77% yield as a white solid using the same method as described for the preparation of hapten **A** (*cf.*Example 3). I.R (KBr): 3118.8; 3034.2; 1700.7; 1613.6; 1515.56;
NMR 13C (CD30D): 169.6; 158.6; 158.5; 144.2; 134.2; 132.9; 131.3(2C); 130.9(2C); 130.5; 128.4(2C); 128.1 (2C); 116.4(2C); 116.1(2C); 70.3; 70.1; 52.3; 52.25; 35.4; 31.8 and 16.5.

**Example 7: Preparation of N-[2-Hydroxy-2-(4-hydroxyphenyl)ethyl] *t*-butylcarboxamide 9.**

To a solution of octopamine hydrochloride **4** (18.96g, 0.1 mole) in methanol (200ml) was added TEA (20.24g, 0.2 mole) and di-tert-butyldicarbonate (26.19g, 0.12 mole). The mixture was stirred at room temperature overnight. Solvents were removed *in vacuo* and the crude residue obtained was purified by flash chromatography on silica gel using ethyl acetate as eluant to give N-BOC octopamine **9** (23,2g, 80%) as a white solid.

**Example 8: Preparation of Ethyl 4-{4-[2-(t-butylcarboxamido)-1-hydroxyethyl]phenoxy}butanoate 10.**

To a solution of N-BOC octopamine **9** (23.2g, 0.08 mole) in acetonitrile (400ml) was added potassium carbonate (44.29g, 0.32 mole) and ethyl bromobutyrate (23.41g, 0.12 mole). The mixture was stirred and heated at reflux overnight. The solution was then allowed to cool to room temperature, filtered and concentrated to dryness. The residue obtained was purified by flash chromatography on silica gel using 50% ethyl acetate in hexane as eluant to give the title compound 10 (24.53g, 78%) as a clear oil.
I.R(Film): 3434.2; 2979.7; 2934.5; 1731.3(broad); 1611.9; 1512.5; 1367.5; 1247.2 and 1172.8.

**Example 9: Preparation of Ethyl 4-[4-(2-amino-1-hydroxyethyl)phenoxy]butanoate trifluoroacetic acid salt 11.**

To a solution of ethyl 4-(carboxypropyl)-N-BOC octopamine **10** (14.26g, 0.0363 mole) in dichloromethane (80ml) was added trifluoroacetic acid (40ml) and the mixture was stirred at room temperature for 1 hour. The solvents were removed *in vacuo* and the crude product obtained was purified by flash chromatography on silica gel using (10% methanol in chloroform) to give the title compound **11** (12.2g, 83%) as a viscous oil.

**Example 10: Preparation of 10'-O-[3-(Ethoxycarbonyl)propyl]ractopamine ether 12.**

The ester **12** was prepared by condensation of compound **11** (21.89g, 0.0574 mole) with 4-(4-hydroxyphenyl)-2-butanone, **2** (9.43g, 0.0574 mole) using the same method as described for the preparation of **5** and **8**. The title compound **12** (10g, 42%) was obtained as a yellow oil.
I. R(Film): 3297.7(broad); 2936.7; 1732.1; 1612.07; 1514.5; 1446.4; 1375.5; 1174.3 and 1029.7

**Example 11: Preparation of 10'-O-(3-Carboxypropyl)ractopamine ether hapten C.**

The hapten **C** was obtained after saponification of the ester **12,** using the same method as described for the preparation of haptens **A** and **B,** as a white solid (6.2g, 66%).

NMR 13C (CD30D): 177.17; 160.46; 156.9; 134.3; 132.4; 130.4(2C); 128.4(2C); 116.4(2C), 115.7(2C); 70.0; 68.1, 55.4; 52.2; 35.6; 31.7; 31.5; 25.8 and 16.5

**Example 12: Preparation of Isoxsuprine 15.**

To a solution of α-(1-aminoethyl)-4-hydroxybenzyl alcohol **13** (10.0g, 0.049 mole) and phenoxy-2-propanone **14** (7.4g, 0.049 mole) in methanol (200ml) was added TEA (10.5g, 0.102 mole). The mixture was stirred at room temperature overnight. Solvents were removed *in vacuo.* To the residue were added HCl (1N) (100ml) and water (100ml) and this was extracted with ethyl acetate (1 x 200ml). The aqueous phase was made basic (pH 12-13) using sodium hydroxide (6N) and extracted with ethyl acetate (3 x 200ml). The organic extracts were combined and washed with brine (1 x 200ml), dried over sodium sulfate, filtered and evaporated to dryness. The crude product was purified by flash chromatography on silica gel using 10 % methanol in chloroform to give isoxsuprine **15** (8g, 54%) as a white solid, m.p. 102-104°C.

**Example 13: Preparation of 10'-O-(4-tert-Butoxycarbonylmethyl) isoxsuprine ether 16.**

Isoxsuprine, **15** (3.1g, 10.3mMoles) was dissolved in acetonitrile (100ml). To this solution was added potassium carbonate (4.48g, 32.4mMoles) and tert-butyl bromoacetate (3.16g, 16.2mMoles) and the mixture heated at reflux overnight. The mixture was allowed to cool, filtered and the filtrate concentrated *in vacuo.* The crude product so obtained was purified by flash chromatography on silica gel using 5 % methanol in chloroform to give **16** (2.27g, 52%) as a colourless oil. NMR $^{13}C$ ($CDCl_3$): 168.1, 158.7, 157.0, 134.5, 128.5, 127.3, 120.9, 114.5, 83.3, 73.7, 71.9, 65.9, 56.0, 49.6, 28.0, 18.3 and 15.2.

**Example 14: Preparation of 10'-O-(4-Carboxymethyl)isoxsuprine ether, Hapten D**

To a solution of 10'-O-(4-tert-butoxycarbonylmethyl)isoxsuprine ether **16** (2.08g, 4.8mMoles) in dichloromethane (25ml) was added trifluoroacetic acid (1 0ml) and the mixture was stirred at room temperature for two hours. The solvent was removed *in vacuo* and the crude product obtained was purified by flash chromatography on silica gel using 10% methanol in chloroform to give the Hapten **D** as a TFA salt (1.5g, 66%).

**Example 15: Preparation of tert-Butyl 4-[2-hydroxyethyl]phenoxyacetate 18.**

To a solution of 2-(4-hydroxyphenyl) ethanol **17** (27.6g, 0.2 mole) in acetonitrile (300ml) was added potassium carbonate (110.5g, 0.8mole) and tert-butyl bromo acetate (46.8g, 0.24mole) and the mixture was stirred and heated at reflux for two hours. The mixture was allowed to cool, filtered and the filtrate concentrated *in vacuo.* The crude product obtained was purified by chromatography on silica gel by using 30% ethyl acetate in hexane to tert-butyl 4-[2-hydroxyethyl] phenoxyacetate **18** (39.8g, 83%) as a clear oil.

**Example 16: Preparation of tert-Butyl 4-[formylmethyl]phenoxyacetate 19.**

To a solution of **18** (20.0g, 0.083mole) in anhydrous dichloromethane (200ml) was added pyridinium chlorochromate (PCC) (44.7g, 0.207mole) and the mixture was stirred at room temperature for two hours. Ether (500ml) was then added to quench the reaction and the mixture was then filtered through C elite. (Trade Mark) Evaporation of the solvents *in vacuo* afforded the crude product which was purified by flash chromatography on silica gel using 20% ethyl acetate in hexane to give **19** (16.6g, 80%) as a yellow oil.

**Example 17: Preparation of t-Butyl 4-[-2-(beta-hydroxy-alpha-methyl-4-hydroxyphenethylamino)ethyl] phenoxyacetate, 20.**

The ester **20** was prepared by condensation of tert-butyl 4-[formylmethyl] phenoxyacetate (10.0g, 0.04mole) with α-(1-aminoethyl)-4-hydroxybenzyl alcohol **13** (8.2g, 0.04mole) using the same method as described for the preparation of **5**, **8,** and **16**. The ester **20** (8.18g, 51%) was obtained as a yellow oil.

**Example 18: Preparation of 4-[-2- (beta-hydroxy-alpha-methyl-4-hydroxyphenethylamino)ethyl]phenoxyethanoic acid, Hapten E**

To a solution of tert-butyl ester **20** (3.7g, 0.92mMole) in 25ml of anhydrous dioxane was added hydrochloric acid solution (4M) in dioxane (25ml) and the mixture was stirred overnight at room temperature. Evaporation of the solvents *in vacuo* afforded the crude product which was purified by flash chromatography on silica gel using 10% methanol in chloroform to give the hapten **E** HCl salt.(1.2g, 34.2%) as a foamy solid.

NMR$^{13}$C ($CD_3OD$): 171.4, 159.6,158.6, 132.6 (2), 131.6 (2), 130.8 (2), 128.3 (2), 116.6, 116.1, 84.8, 66.8, 59.8, 52.7, 32.6 and 19.4.

**Example 19: Conjugation of Haptens A, B, C, D and E to BSA (Preparation of Immunogens A, B, C, D and E).**

To a solution of hapten **A, B, C, D** or **E** (58mg, 0.15 mmole) in DMF(1ml) was added N,N-dicyclohexylcarbodiimide (DCC) (34mg, 0.165mmole) and N-hydroxysuccinimide (19mg, 0.165mmole) and the mixture stirred at room trmperature overnight. The dicyclohexylurea formed was removed by filtration and the filtrate added drop-wise to a solution of BSA (200mg) in 0.1M sodium hydrogen carbonate, pH8.5 (12ml). The mixture was stirred at room temperature overnight. The solution was dialysed against 50mM phosphate buffer, pH7.2 (3 changes) for 24 hours at 4°C and then freeze-dried.

MALDI results showed 17 molecules of hapten **A**, 5.8 molecules of hapten **B**, 10.7 molecules of hapten **C**, 12.4 molecules of Hapten **D** and 6.2 molecules for hapten **E** had been conjugated to 1 molecule of BSA.

**Example 20: General method for the conjugation of Haptens A, B, C, D and E to HRP (Horseradish peroxidase).**

EDC.HCl (10mg) was dissolved in water (0.5ml) and immediately added to a solution of Hapten **(A** or **B** or **C** or **D** or **E)** (2mg) in DMF (0.2ml). After mixing, this solution was added drop-wise to a solution of HRP (20mg) in water (1ml). Sulfo-NHS (5mg) was added and the reaction mixture was incubated in the dark at room temperature overnight. Excess hapten was removed by desalting with PD-10 columns (Pharmacia) in series, pre-equilibrated with PBS (phosphate buffered saline) at pH 7.2. The hapten-HRP conjugate was then dialysed overnight against 10L of PBS pH 7.2 at 4°C.

**Example 21: Preparation of antibodies to Immunogens A and C, prepared in Example 19.**

An aqueous solution of each immunogen was formulated with Freund's Complete Adjuvant (FCA) to form an emulsion consisting of 2mg/ml immunogen in 50% (v/v) FCA. Three sheep were immunised with this emulsion (1° immunisation), 0.25ml being subcutaneously injected at each of four sites in the flank of each animal. Subsequent immunizations (boosts) contained 1mg/ml immunogen. All boosts were emulsified in 50% (v/v) Freund's Incomplete Adjuvant (FIA) and were administered in the same manner as the 1 ° immunisation, at monthly intervals for 1 year. Blood sampling took place 7 to 14 days after each boost. Each sample was processed to produce antiserum, which was further purified by caprylic acid and ammonium sulfate precipitation to yield an immunoglobulin G (IgG) fraction. The IgG fraction was evaluated by competitive ELISA microtiter plate assay, as described in Example 22 below.

**Example 22: Development of competitive ELISAs for ractopamine.**

a) The wells of an enhanced binding 96 well polystyrene microtiter plate were coated with the IgG fraction of the antiserum raised to immunogen **A** (hapten **A-**BSA) (Example 19), diluted in 10mM Tris, pH8.5 (125μl/well). The appropriate antibody coating dilution was determined using standard ELISA checkerboard techniques. The plate was incubated for 2 hours at 37°C, washed 4 times with Tris buffered saline containing Tween 20 (TBST) and tapped dry. Standard solutions of ractopamine were prepared in TBST at 0, 0.05, 0.1, 0.5, 1, and 5ng/ml, and μl of each was added to the appropriate wells. 75μl of conjugate **A** (hapten **A**-HRP) (Example 20), diluted in Tris buffer (pH 7.2) containing EDTA, D-mannitol, sucrose, thimerosal and BSA, was added to each of the wells. The appropriate dilution of conjugate was also determined using standard ELISA checkerboard techniques. The plate was incubated at 37°C for 2 hours. Excess unbound conjugate was removed by washing 6 times over a 10 minute period with TBST. 125ul of tetramethylbenzidine (TMB) substrate solution was added to each well of the plate that was then incubated for 15 to 20 minutes in the dark at room temperature. The reaction was terminated by addition of 125 μl 0.2M $H_2SO_4$ to each well. The absorbance was then measured at 450nm using a microtiter plate reader. The data generated in the assay is presented in Table 1.

b) In a similar manner to that described in Example 15(a), the wells of a 96-well microtiter plate were coated with the IgG fraction of the antiserum raised to immunogen **C** (hapten **C**-BSA) (Example 19), standards were applied at 0, 0.025, 0.1, 0.25, 0.5 and 1ng/ml and conjugate **C** (hapten **C**-HRP) (Example 20) was employed as detection reagent. The data

generated are presented in Table 1. The same definitions apply for $A_{450}$, B, $B_0$ and $IC_{50}$.

Table 1: Data generated from competitive microtiter plate assays for ractopamine, employing antisera generated to immunogen A (hapten A-BSA) (Example 19) and immunogen C (hapten C-BSA) (Example 19).

| Example 22 a) | | | Example 22b) | | |
|---|---|---|---|---|---|
| **Ractopamine Concentration ng/ml** | **$A_{450}$** | **%B/$B_0$** | **Ractopamine Concentration ng/ml** | **$A_{450}$** | **%B/$B_0$** |
| 0 | 2.385 | 100 | 0 | 1.994 | 100 |
| 0.05 | 1.276 | 53.5 | 0.025 | 1.683 | 84 |
| 0.1 | 1.181 | 49.5 | 0.1 | 1.302 | 65 |
| 0.5 | 0.566 | 23.7 | 0.25 | 0.918 | 46 |
| 1 | 0.404 | 16.9 | 0.5 | 0.68 | 34 |
| 5 | 0.151 | 6.33 | 1 | 0.469 | 24 |
| | | | | | |
| **$IC_{50}$ (ng/ml)** | 0.082 | | **$IC_{50}$ (ng/ml)** | 0.202 | |
| $A_{450}$ = absorbance at 450nm<br>B = absorbance at 450nm at xng/ml standard concentration<br>$B_0$ = absorbance at 450nm at 0ng/ml standard concentration<br>$IC_{50}$ = standard concentration which produces 50% B/$B_0$<br>%CR = percentage cross-reactivity based on specificity to ractopamine - this is what is meant by the term "cross-reactivity" in the present specification. | | | | | |

**Example 23: Cross reactivity of Competitive ELISAs for Ractopamine**

In order to determine the specificity of the competitive ELISAs for ractopamine, standard solutions of a range of structurally similar β-agonists were prepared in TBST. Employing each series of standards in the ractopamine competitive ELISAs, calibration curves were generated and these were used to determine the cross-reactivity of each immunoassay with these substances. The results of this study are presented in Table 2, cross-reactivity being calculated according to the following formula:

$$\%CR = IC_{50,\ \mathrm{ractopamine}} / IC_{50,\ CR} \times 100$$

Where %CR is the percentage cross-reactivity, $IC_{50,\ \mathrm{ractopamine}}$ is the concentration of ractopamine that causes 50% displacement of signal and $IC_{50,\ CR}$ is the concentration of potential cross-reactant that causes 50% displacement of signal.

**Table 2:** Cross reactivity of the competitive ELISAs for ractopamine

| **Compound** | **Example 23 a)** | | **Example 23 b)** | | **Shelver pAb** | | **Shelver mAb** | |
|---|---|---|---|---|---|---|---|---|
| | **$IC_{50\ CR}$ ng/ml** | **%CR** | **$IC_{50\ CR}$ ng/ml** | **%CR** | **$IC_{50\ CR}$ ng/ml** | **%CR** | **$IC_{50\ CR}$ ng/ml** | **%CR** |
| Ractopamine | 0.082 | 100 | 0.202 | 100 | 4.2 | 100 | 2.69 | 100 |
| Dobutamine | 44.2 | 0.186 | >50 | <0.8 | 12,7 | 33 | 50.3 | 5.3 |
| Isoxsuprine | >50 | <0.16 | >50 | <0.6 | 6,200 | 0.1 | 1,391 | 0.2 |
| DL-Isoproterenol | >50 | <0.16 | >50 | <0.5 | >100,000 | <0.01 | NA | NA |

(continued)

| Compound | Example 23 a) | | Example 23 b) | | Shelver pAb | | Shelver mAb | |
|---|---|---|---|---|---|---|---|---|
| | $IC_{50\ CR}$ ng/ml | %CR | $IC_{50\ CR}$ ng/ml | %CR | $IC_{50\ CR}$ ng/ml | %CR | $IC_{50\ CR}$ ng/ml | %CR |
| Ritodrine | >50 | <0.16 | >50 | <0.5 | 577 | 0.8 | 73.8 | 3.6 |
| Salmeterol Xinafoate | >50 | <0.16 | >50 | <0.5 | 1,800 | 0.2 | 1519 | 0.2 |
| Methyl Clenbuterol | >50 | <0.16 | >50 | <0.05 | NA | NA | NA | NA |
| Fenoterol | >50 | <0.16 | >50 | <0.05 | 310 | 1.3 | 2,682 | 0.1 |
| Pirbuterol | >50 | <0.16 | >50 | <0.05 | NA | NA | NA | NA |
| Tulobuterol | >50 | <0.16 | >50 | <0.05 | NA | NA | NA | NA |
| Dopamine | >50 | <0.16 | >50 | <0.01 | NA | NA | NA | NA |
| Salbutamol | >50 | <0.16 | >50 | <0.01 | 79,000 | <0.01 | NA | <0.1 |
| Clenbuterol | >50 | <0.16 | >50 | <0.01 | >100,000 | <0.01 | NA | <0.1 |
| Metaproterenol | >50 | <0.16 | >50 | <0.01 | >100,000 | <0.01 | NA | NA |
| Terbutaline | >50 | <0.16 | >50 | <0.01 | NA | NA | NA | NA |
| Cimaterol | >50 | <0.16 | >50 | <0.01 | NA | NA | NA | NA |
| Bamethane | >50 | <0.16 | >50 | <0.01 | 2,900 | 0.1 | 831 | 0.3 |
| (-) Isoproterenol | >50 | <0.16 | >50 | <0.01 | NA | NA | NA | NA |
| Mabuterol | >50 | <0.16 | >50 | <0.01 | NA | NA | NA | NA |

$IC_{50,CR}$ = concentration of potential cross-reactant that causes 50% displacement of signal
%CR = percentage cross-reactivity based on specificity to ractopamine
Shelver pAb = Shelver polyclonal antiserum (Shelver and Smith, Journal of Immunoassay (2000), 21(1))
Shelver mAb = Shelver monoclonal antibody (Shelver et al., J. Agric. Food Chem. 48 (2000))

The data presented in Table 2 indicate that the ractopamine immunoassays developed employing antibodies generated to hapten **A** and hapten **B** are highly sensitive with $IC_{50}$ values of 0.082 and 0.202ng/ml, respectively. These immunoassays are considerably more sensitive than those presented in the prior art. For example, Shelver et al. (2000) reported an $IC_{50}$ of 4.2ng/ml for their ractopamine EIA. The specificity data presented in Table 2 indicate that the antibodies employed are highly specific for ractopamine. There is negligible cross-reactivity with dobutamine (<0.8%) for both antibodies in the present application, compared with that published in the prior art. For example, Shelver et al reported 33% cross-reactivity with dobutamine for their polyclonal antibody and 5.3% cross-reactivity with dobutamine for their monoclonal antibody.

The ractopamine-derived antibodies from the present invention will therefore yield highly specific results when screening biological samples for the presence of ractopamine.

**Example 24: Development of a Competitive ELISA for Isoxsuprine**

In a similar manner to that described in Example 22(a), the wells of a 96-well microtiter plate were coated with the IgG fraction of the antiserum raised to immunogen **D** (hapten **D**-BSA) (Example 19), isoxsuprine standards were applied at 0, 0.5, 1, 5, 10, 50, 100 and 200ng/ml and conjugate **D** (hapten **D**-HRP) (Example 20) was employed as detection reagent. The data generated are presented in Table 3.

**Example 25: Development of a Competitive ELISA for Ritodrine**

In a similar manner to that described in Example 22(a), the wells of a 96-well microtiter plate were coated with the IgG fraction of the antiserum raised to immunogen **E** (hapten **E**-BSA) (Example 19), ritodrine standards were applied

at 0, 1, 5, 10, 50, 100, 500 and 1000ng/ml and conjugate **E** (hapten **E**-HRP) (Example 20) was employed as detection reagent. The data generated are also presented in Table 3.

**Table 3**: Data generated from competitive microtiter plate assays for isoxsuprine and ritodrine, employing antisera generated to immunogen **D** (hapten **D**-BSA) (Example 19) and immunogen **E** (hapten **E**-BSA) (Example 19).

| Example 24 | | | Example 25 | | |
|---|---|---|---|---|---|
| **Isoxsuprine Concentration ng/ml** | **$A_{450}$** | **%B/$B_0$** | **Ritodrine Concentration ng/ml** | **$A_{450}$** | **%B/$B_0$** |
| 0 | 2.345 | 100 | 0 | 1.946 | 100 |
| 0.5 | 0.743 | 31.6 | 1 | 1.791 | 92.0 |
| 1 | 0.399 | 17.0 | 5 | 1.602 | 82.3 |
| 5 | 0.158 | 6.7 | 10 | 1.562 | 80.2 |
| 10 | 0.115 | 4.9 | 50 | 1.260 | 64.7 |
| 50 | 0.036 | 1.5 | 100 | 1.088 | 55.9 |
| 100 | 0.032 | 1.3 | 500 | 0.584 | 30.0 |
| 200 | 0.022 | 0.9 | 1000 | 0.440 | 22.6 |
| | | | | | |
| **$IC_{50}$ (ng/ml)** | 0.230 | | **$IC_{50}$ (ng/ml)** | 115.230 | |

$A_{450}$ = absorbance at 450nm
B = absorbance at 450nm at xng/ml standard concentration
$B_0$ = absorbance at 450nm at 0ng/ml standard concentration
$IC_{50}$ = standard concentration which produces 50% B/$B_0$

**Example 26: Cross reactivity of Competitive ELISAs for Isoxsuprine and Ritodrine**

In a similar manner to that described in Example 23, the cross reactivity of each immunoassay with isoxsuprine, ritodrine, dobutamine and ractopamine was determined. The results of this study are presented in Table 4.

**Table 4:** Cross reactivity of the competitive ELISAs for isoxsuprine (Example 24) and ritodrine (Example 25)

| **Compound** | **Example 24** | | **Example 25** | |
|---|---|---|---|---|
| | **$IC_{50\ CR}$ ng/ml** | **%CR** | **$IC_{50\ CR}$ ng/ml** | **%CR** |
| Isoxsuprine | 0.230 | 100 | 569.7 | 20.2 |
| Ritodrine | >200 | <0.1 | 115.2 | 100 |
| Dobutamine | >200 | <0.1 | >1000 | <5.0 |
| Ractopamine | >200 | <0.1 | > 1000 | <0.5 |

**$IC_{50\ CR}$ =** Concentration of potential cross-reactant that causes 50% displacement of signal
**%CR** = Percentage cross-reactivity based on specificity to isoxsuprine (Example 24) or ritodrine (Example 25)

This high degree of specificity shown in Examples 23 and 26 has been achieved by targeted derivatisation at a single phenolic hydroxy group of phenethanolamines such as, but not limited to, ractopamine, isoxsuprine and ritodrine and ensures that false positive results are kept to a minimum.

## Claims

**1.** A method of preparing a hapten of formula I

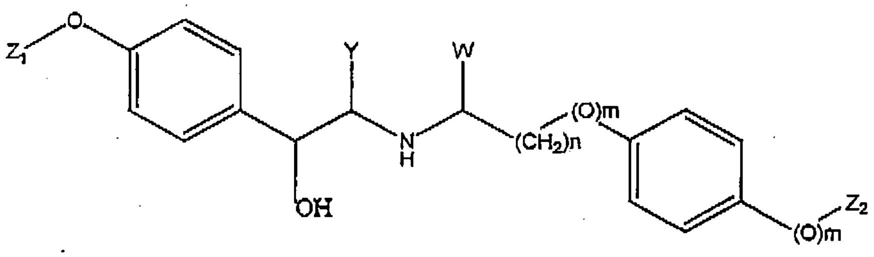

**Hapten I**

suitable for use in raising an antibody capable of binding to at least one structural epitope of a phenethanolamine such as, but not limited to, ractopamine, isoxsuprine or ritodrine, the method comprising the step of reacting a phenethanolamine derivative of the formula D, in which $Z_1$ is a crosslinker, with a phenylalkylcarbonyl derivative of the formula E, in which $Z_2$ is H; Y is independently H or $CH_3$; W is independently H or $CH_3$; n is independently 1 or 2; and m is independently 0 or 1

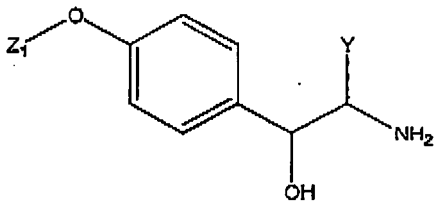

D

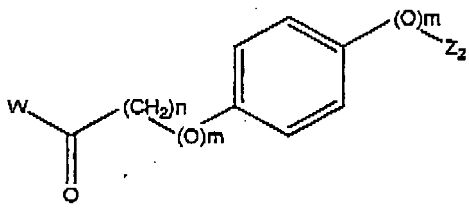

E

2. A method according to claim 1, in which the phenethanolamine derivative of the formula D is prepared by reacting octopamine hydrochloride with $Z_1X_1$, in which $X_1$ is a halide, preferably a bromide.

3. A method according to claim 2, in which the amine group of the octapamine hydrochloride is temporarily protected with BOC, by reacting the octapamine hydrochloride with di-tert-butyldicarbonate before reacting the BOC-protected octapamine hydrochloride with the $Z_1X_1$, the BOC group being subsequently removed.

4. A method of preparing a hapten of formula II

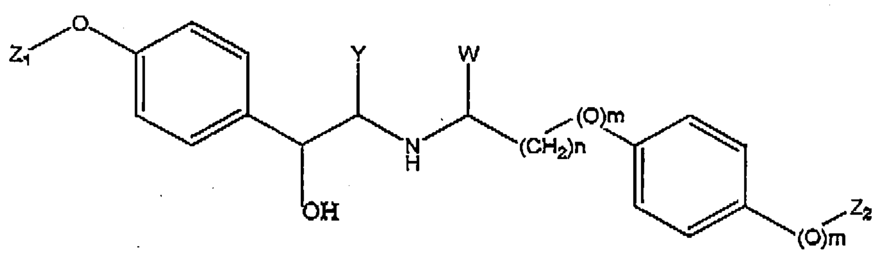

**Hapten II**

suitable for use in raising an antibody capable of binding to at least one structural epitope of a phenethanolamine such as, but not limited to, ractopamine, isoxsuprine or ritodrine, the method comprising the step of reacting a phenethanolamine derivative of the formula D, in which $Z_1$ is H, with a phenylalkylcarbonyl derivative of the formula

E, in which $Z_2$ is a crosslinker; Y is independently H or $CH_3$; W is independently H or $CH_3$; n is independently 1 or 2; and m is independently 0 or 1

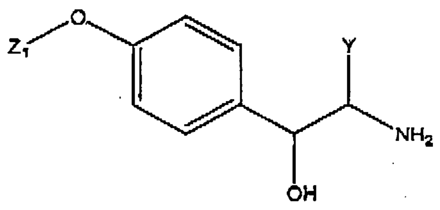

D

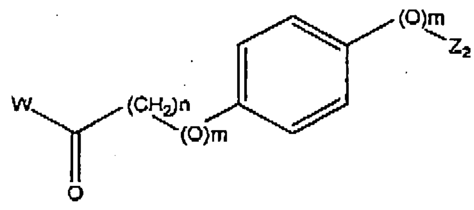

E

5. A method according to claim 4, in which the phenylalkylcarbonyl derivative of the formula E is prepared by reacting 4-(4-hydroxyphenyl)-2-butanone with $Z_2X_1$, in which $X_1$ is a halide, preferably a bromide.

6. A method of making an immunogen, the method comprising preparing a hapten in accordance with the method of any one of claims 1 to 3; and coupling the hapten to an antigenicity-conferring carrier material, the carrier material being preferably selected from the group comprising a protein, a protein fragment, a synthetic polypeptide and a semi-synthetic polypeptide.

7. A method of making an immunogen, the method comprising preparing a hapten in accordance with the method of claim 4 or 5; and coupling the hapten to an antigenicity-conferring carrier material, the carrier material being preferably selected from the group comprising a protein, a protein fragment, a synthetic polypeptide and a semi-synthetic polypeptide.

8. A method of making an antibody, the method comprising preparing an immunogen in accordance with the method of claim 6 and immunising a non-human animal with the immunogen, the antibody being capable of binding to at least one structural epitope of a phenethanolamine such as ractopamine, isoxsuprine or ritodrine.

9. A method of making an antibody, the method comprising preparing an immunogen in accordance with the method of claim 7 and immunising a non-human animal with the immunogen, the antibody being capable of binding to at least one structural epitope of a phenethanolamine such as ractopamine, isoxsuprine or ritodrine.

10. A method of making a conjugate, the method comprising preparing a hapten in accordance with the method of any one of claims 1 to 3; and covalently bonding the hapten to a detectable labelling agent, the labelling agent being preferably selected from the group comprising an enzyme, a luminescent substance, a chemiluminescent substance and a radioactive substance.

11. A method of making a conjugate, the method comprising preparing a hapten in accordance with the method of claim 4 or 5; and covalently binding the hapten to a detectable labelling agent, the labelling agent being preferably selected from the group comprising an enzyme, a luminescent substance, a chemiluminescent substance and a radioactive substance.

**Patentansprüche**

1. Verfahren zum Herstellen eines Haptens der Formel I

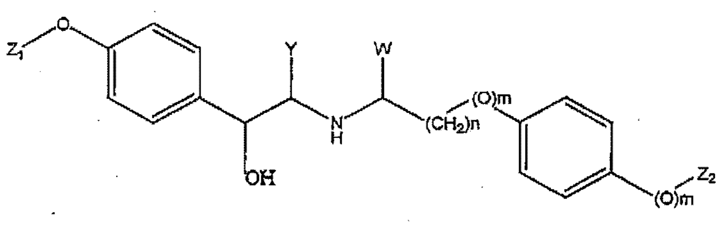

**Hapten I**

das zur Verwendung beim Anheben eines Antikörpers geeignet ist, der sich an wenigstens ein strukturelles Epitop eines Phenethanolamins wie z.B., aber ohne Begrenzung, Ractopamin, Isoxsuprin oder Ritodrin binden kann, wobei das Verfahren den Schritt des Reagierens eines Phenethanolaminderivats der Formel D, wobei $Z_1$ ein Vernetzer ist, mit einem Phenylalkylcarbonylderivat der Formel E beinhaltet, wobei $Z_2$ H ist; Y unabhängig H oder $CH_3$ ist; W unabhängig H oder $CH_3$ ist; n unabhängig 1 oder 2 ist; und m unabhängig 0 oder 1 ist

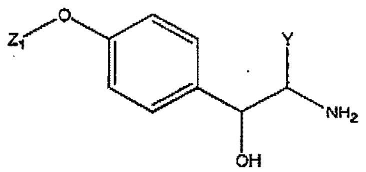

D

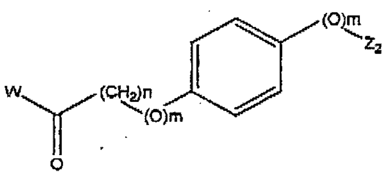

E

2. Verfahren nach Anspruch 1, wobei das Phenethanolaminderivat der Formel D hergestellt wird durch Reagieren von Octopaminhydrochlorid mit $Z_1X_1$, wobei $X_1$ ein Halogenid, vorzugsweise ein Bromid, ist.

3. Verfahren nach Anspruch 2, wobei die Amingruppe des Octapaminhydrochlorids vorübergehend mit BOC geschützt wird, indem das Octapaminhydrochlorid mit Di-tert-butyldicarbonat reagiert wird, bevor das BOC-geschützte Octapaminhydrochlorid mit $Z_1X_1$ reagiert wird, wobei die BOC-Gruppe anschließend entfernt wird.

4. Verfahren zum Herstellen eines Haptens der Formel II

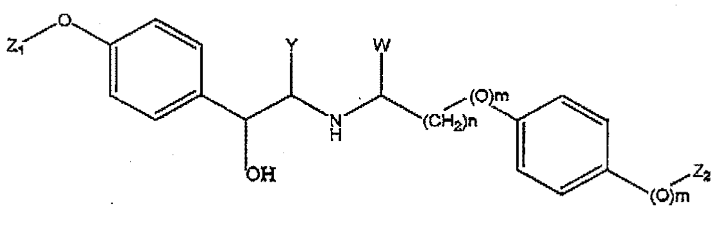

**Hapten II**

das zur Verwendung beim Anheben eines Antikörpers geeignet ist, der sich an wenigstens ein strukturelles Epitop eines Phenethanolamins wie z.B., aber ohne Begrenzung, Ractopamin, Isoxsuprin oder Ritodrin binden kann, wobei das Verfahren den Schritt des Reagierens eines Phenethanolaminderivats der Formel D, wobei $Z_1$ H ist, mit einem Phenylalkylcarbonylderivat der Formel E beinhaltet, wobei $Z_2$ ein Vernetzer ist; Y unabhängig H oder $CH_3$ ist; W

unabhängig H oder $CH_3$ ist; n unabhängig 1 oder 2 ist; und m unabhängig 0 oder 1 ist

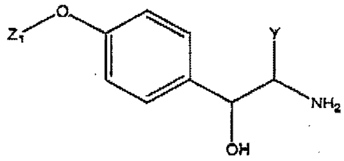

D

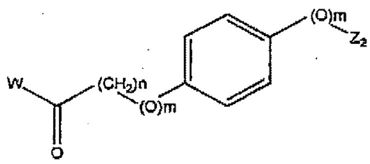

E

5. Verfahren nach Anspruch 4, wobei das Phenylalkylcarbonylderivat der Formel E hergestellt wird durch Reagieren von 4-(4-Hydroxyphenyl)-2-butanon mit $Z_2X_1$, wobei $X_1$ ein Halogenid, vorzugsweise ein Bromid, ist.

6. Verfahren zum Erzeugen eines Immunogens, wobei das Verfahren das Herstellen eines Haptens gemäß dem Verfahren nach einem der Ansprüche 1 bis 3; und Koppeln des Haptens an ein Antigenität verleihendes Trägermaterial beinhaltet, wobei das Trägermaterial vorzugsweise ausgewählt wird aus der Gruppe umfassend ein Protein, ein Proteinfragment, ein synthetisches Polypeptid und ein halbsynthetisches Polypeptid.

7. Verfahren zum Erzeugen eines Immunogens, wobei das Verfahren das Herstellen eines Haptens gemäß dem Verfahren nach Anspruch 4 oder 5; und Koppeln des Haptens an ein Antigenität verleihendes Trägermaterial beinhaltet, wobei das Trägermaterial vorzugsweise ausgewählt wird aus der Gruppe umfassend ein Protein, ein Proteinfragment, ein synthetisches Polypeptid und ein halbsynthetisches Polypeptid.

8. Verfahren zum Erzeugen eines Antikörpers, wobei das Verfahren das Herstellen eines Immunogens gemäß dem Verfahren nach Anspruch 6 und Immunisieren eines nicht-menschlichen Tieres mit dem Immunogen beinhaltet, wobei sich der Antikörper an wenigstens ein strukturelles Epitop eines Phenethanolamins wie Ractopamin, Isoxsuprin oder Ritodrin binden kann.

9. Verfahren zum Erzeugen eines Antikörpers, wobei das Verfahren das Herstellen eines Immunogens gemäß dem Verfahren nach Anspruch 7 und Immunisieren eines nicht-menschlichen Tieres mit dem Immunogen beinhaltet, wobei sich der Antikörper an wenigstens ein strukturelles Epitop eines Phenethanolamins wie Ractopamin, Isoxsuprin oder Ritodrin binden kann.

10. Verfahren zum Erzeugen eines Konjugats, wobei das Verfahren das Herstellen eines Haptens gemäß dem Verfahren nach einem der Ansprüche 1 bis 3; und das kovalente Binden des Haptens an ein detektierbares Markierungsmittel beinhaltet, wobei das Markierungsmittel vorzugsweise ausgewählt wird aus der Gruppe umfassend ein Enzym, eine lumineszierende Substanz, eine chemilumineszierende Substanz und eine radioaktive Substanz.

11. Verfahren zum Erzeugen eines Konjugats, wobei das Verfahren das Herstellen eines Haptens gemäß dem Verfahren nach Anspruch 4 oder 5; und das kovalente Binden des Haptens an ein detektierbares Markierungsmittel beinhaltet, wobei das Markierungsmittel vorzugsweise ausgewählt wird aus der Gruppe umfassend ein Enzym, eine lumineszierende Substanz, eine chemilumineszierende Substanz und eine radioaktive Substanz.

## Revendications

1. Méthode de préparation d'un haptène de formule 1

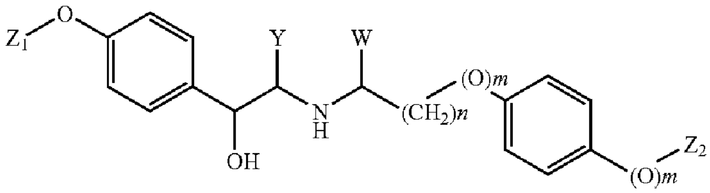

**Haptène I**

convenable pour une utilisation dans la production d'un anticorps capable de se fixer à au moins un épitope structurel d'une phénéthanolamine telle que, sans y être limitée, la ractopamine, l'isoxsuprine ou la ritodrine, la méthode comprenant l'étape consistant à faire réagir un dérivé de phénéthanolamine de formule D, dans laquelle $Z_1$ est un agent de réticulation, avec un dérivé de phénylalkylcarbonyle de formule E, dans laquelle $Z_2$ est H ; Y est indépendamment H ou $CH_3$ ; W est indépendamment H ou $CH_3$ ; n vaut indépendamment 1 ou 2 ; et m vaut indépendamment 0 ou 1

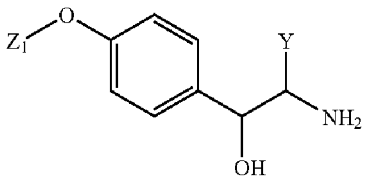

D

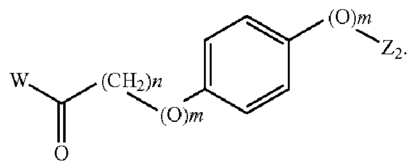

E

2. Méthode selon la revendication 1, dans laquelle le dérivé de phénéthanolamine de formule D est préparé par la réaction de chlorhydrate d'octopamine avec $Z_1X_1$, où $X_1$ est un halogénure, préférablement un bromure.

3. Méthode selon la revendication 2, dans laquelle le groupement amine du chlorhydrate d'octopamine est temporairement protégé par BOC, par la réaction du chlorhydrate d'octopamine avec du dicarbonate de di-tertio-butyle avant la réaction du chlorhydrate d'octopamine protégé par BOC avec $Z_1X_1$, le groupement BOC étant ensuite éliminé.

4. Méthode de préparation d'un haptène de formule II

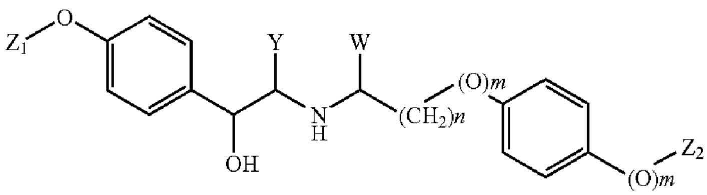

**Haptène II**

convenable pour une utilisation dans la production d'un anticorps capable de se fixer à au moins un épitope structurel d'une phénéthanolamine telle que, sans y être limitée, la ractopamine, l'isoxsuprine ou la ritodrine, la méthode comprenant l'étape consistant à faire réagir un dérivé de phénéthanolamine de formule D, dans laquelle $Z_1$ est H, avec un dérivé de phénylalkylcarbonyle de formule E, dans laquelle $Z_2$ est un agent de réticulation ; Y est indépendamment H ou $CH_3$ ; W est indépendamment H ou $CH_3$ ; n vaut indépendamment 1 ou 2 ; et m vaut indépendamment 0 ou 1

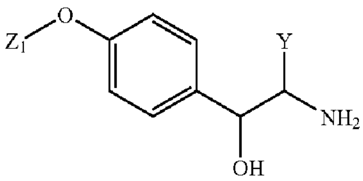

D

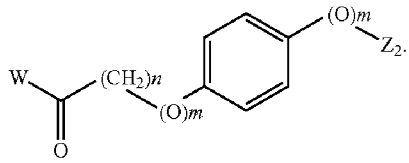

E

5. Méthode selon la revendication 4, dans laquelle le dérivé de phénylalkylcarbonyle de formule E est préparé par la réaction de 4-(4-hydroxyphényl)-2-butanone avec $Z_2X_1$, où $X_1$ est un halogénure, préférablement un bromure.

6. Méthode de production d'un immunogène, la méthode comprenant la préparation d'un haptène conformément à la méthode selon l'une quelconque des revendications 1 à 3 ; et le couplage de l'haptène à un matériau véhicule conférant une antigénicité, le matériau véhicule étant préférablement choisi dans le groupe comprenant une protéine, un fragment de protéine, un polypeptide synthétique et un polypeptide semi-synthétique.

7. Méthode de production d'un immunogène, la méthode comprenant la préparation d'un haptène conformément à la méthode selon la revendication 4 ou 5 ; et le couplage de l'haptène à un matériau véhicule conférant une antigénicité, le matériau véhicule étant préférablement choisi dans le groupe comprenant une protéine, un fragment de protéine, un polypeptide synthétique et un polypeptide semi-synthétique.

8. Méthode de production d'un anticorps, la méthode comprenant la préparation d'un immunogène conformément à la méthode selon la revendication 6, et l'immunisation d'un animal non humain par l'immunogène, l'anticorps étant capable de se fixer à au moins un épitope structurel d'une phénéthanolamine telle que la ractopamine, l'isoxsuprine ou la ritodrine.

9. Méthode de production d'un anticorps, la méthode comprenant la préparation d'un immunogène conformément à la méthode selon la revendication 7, et l'immunisation d'un animal non humain par l'immunogène, l'anticorps étant capable de se fixer à au moins un épitope structurel d'une phénéthanolamine telle que la ractopamine, l'isoxsuprine ou la ritodrine.

10. Méthode de production d'un conjugué, la méthode comprenant la préparation d'un haptène conformément à la méthode selon l'une quelconque des revendications 1 à 3 ; et la fixation covalente de l'haptène à un agent de marquage détectable, l'agent de marquage étant préférablement choisi dans le groupe comprenant une enzyme, une substance luminescente, une substance chimio-luminescente, et une substance radioactive.

11. Méthode de production d'un conjugué, la méthode comprenant la préparation d'un haptène conformément à la méthode selon la revendication 4 ou 5 ; et la fixation covalente de l'haptène à un agent de marquage détectable, l'agent de marquage étant préférablement choisi dans le groupe comprenant une enzyme, une substance luminescente, une substance chimio-luminescente, et une substance radioactive.

## Chemical structures of Ractopamine, Ritodrine and Isoxsuprine

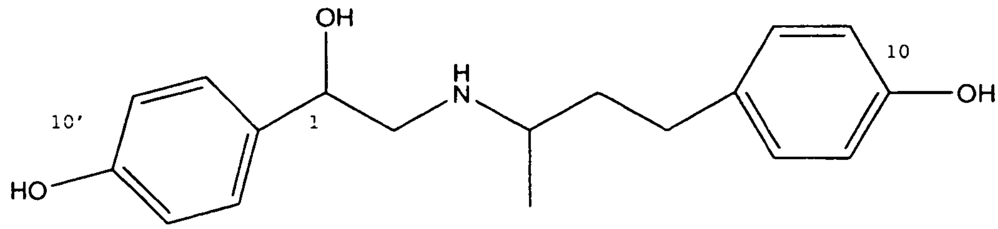

**Ractopamine**

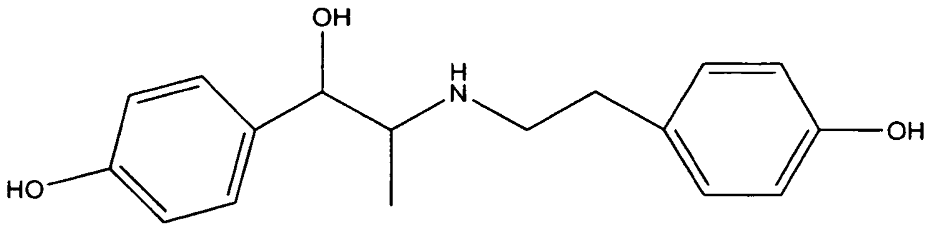

**Ritodrine**

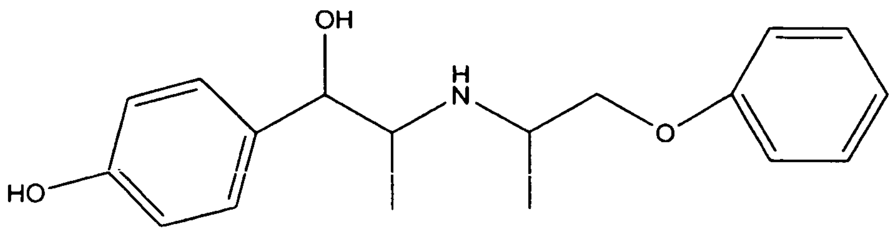

**Isoxsuprine**

**Figure - 1**

## Chemical Structure of Haptens A, B, C, D, and E

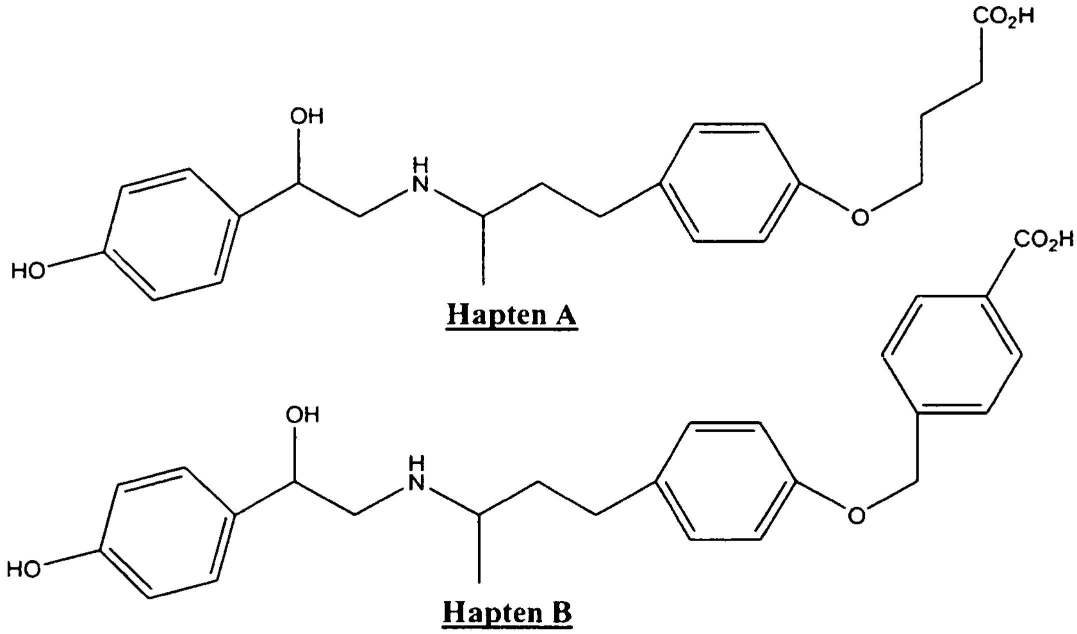

Hapten A

Hapten B

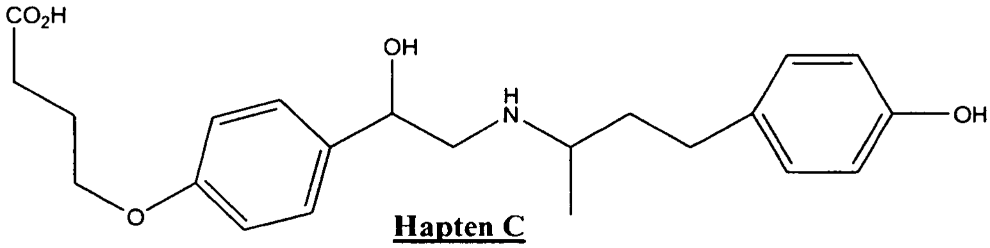

Hapten C

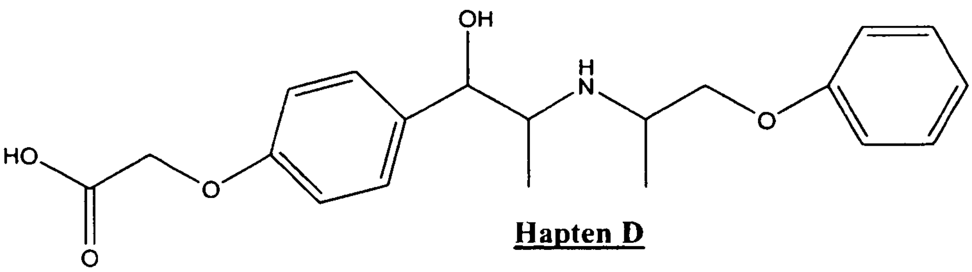

Hapten D

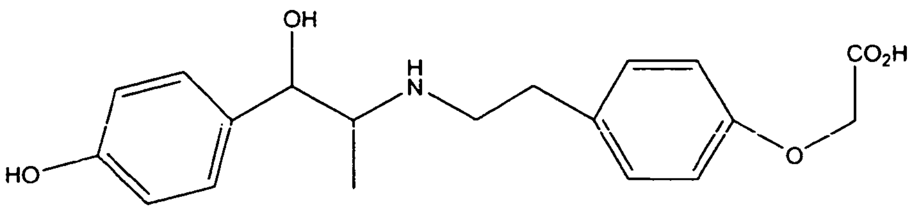

Hapten E

Figure - 2

## Chemical reactions for the preparation of Hapten A and Immunogen A

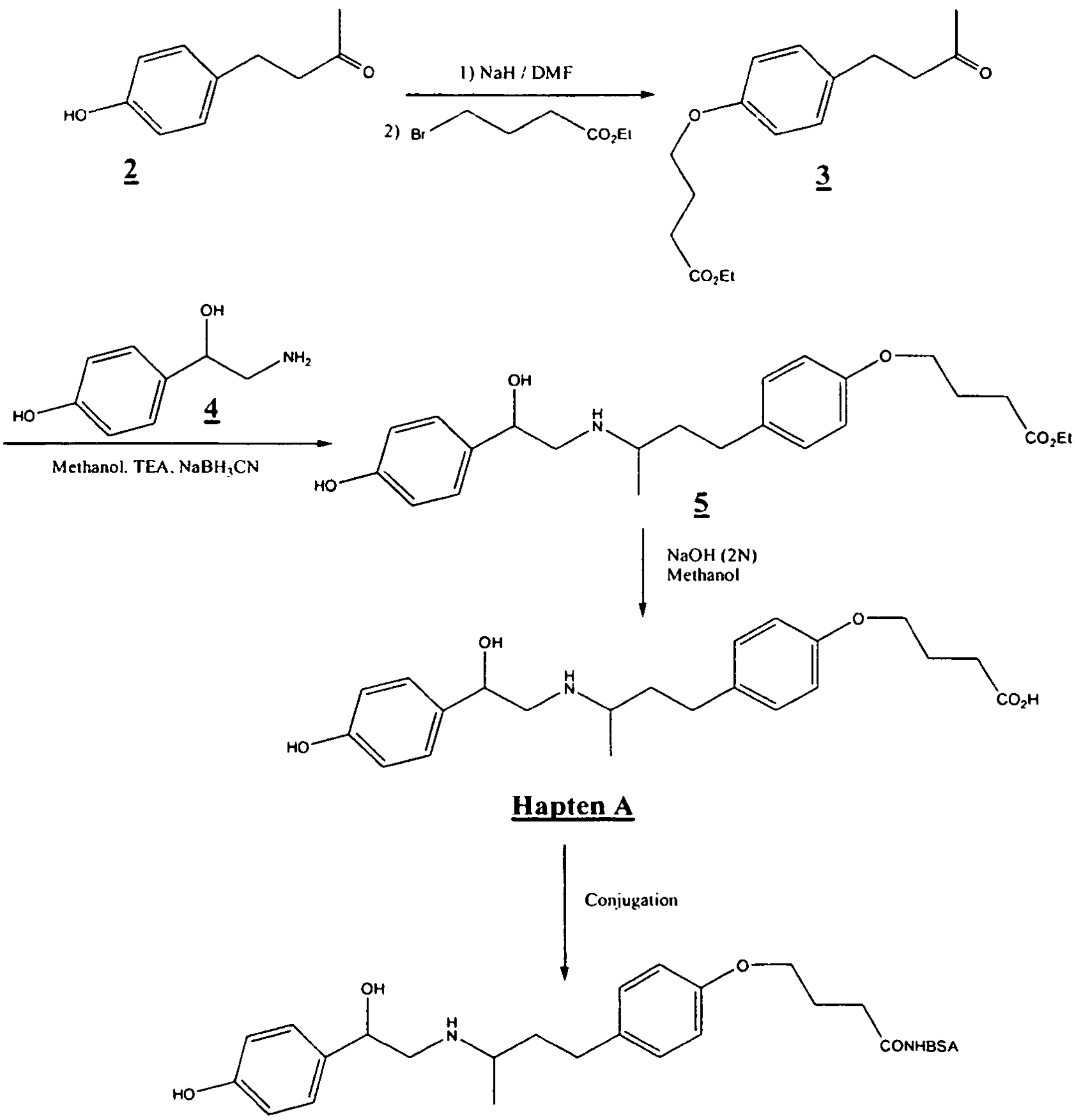


2

3

4

5

**Hapten A**

**Immunogen A**

Figure - 3

## Chemical reactions for the preparation of Hapten B and Immunogen B

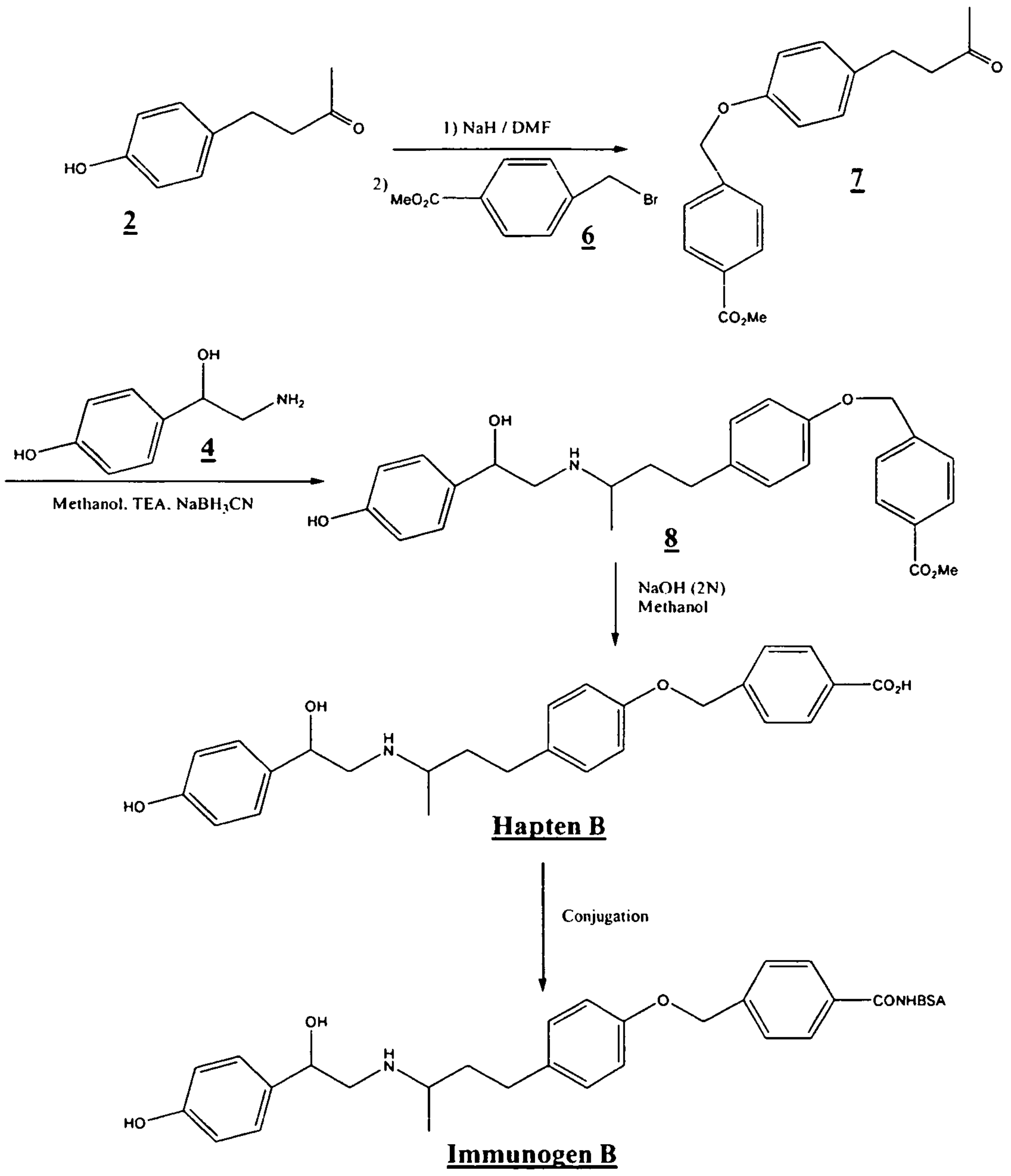


2

6

7

4

8

Hapten B

Immunogen B

**Figure - 4**

## Chemical reactions for the preparation of Hapten C and Immunogen C

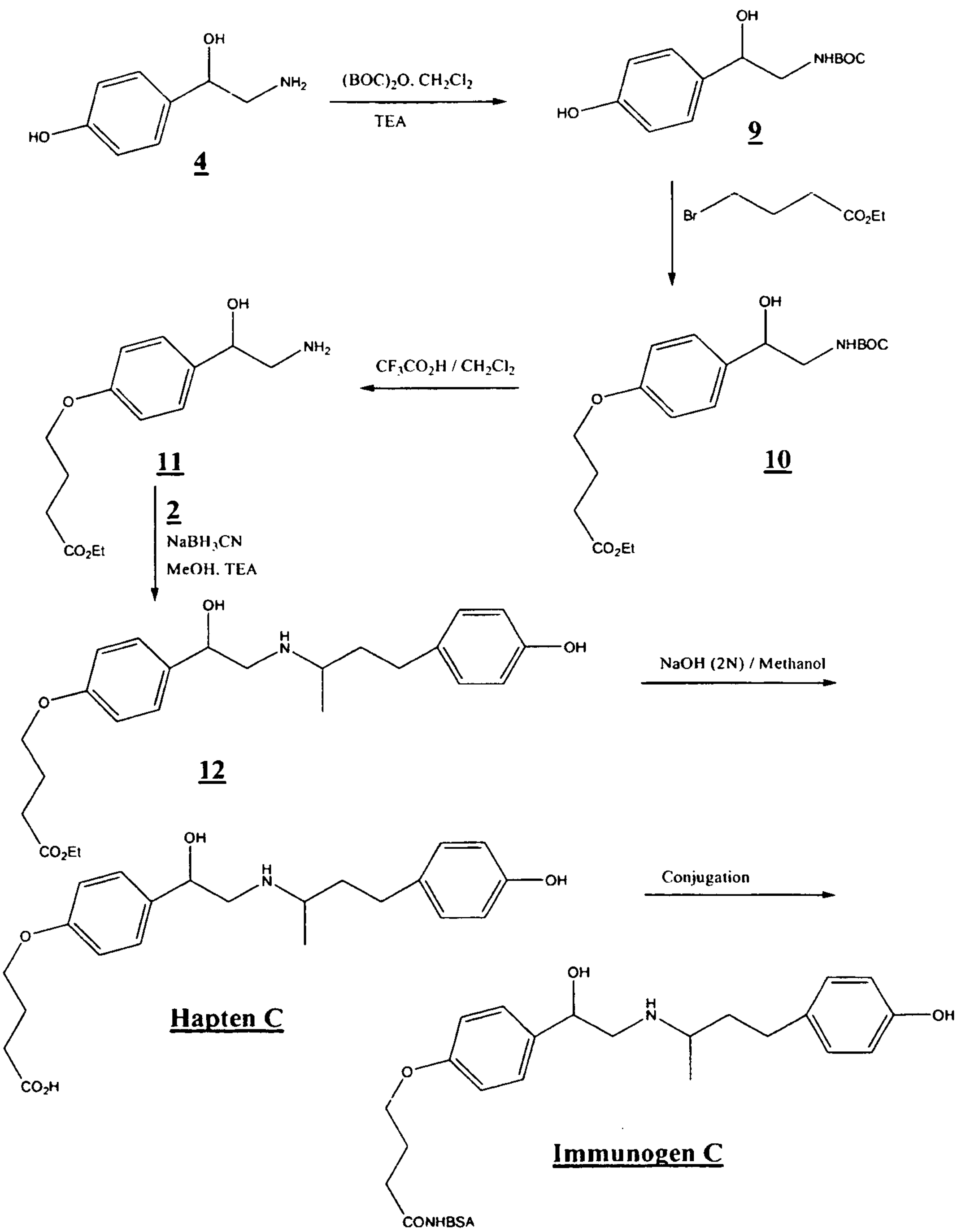


4

9

10

11

12

Hapten C

Immunogen C

Figure - 5

## Chemical reactions for the preparation of Hapten D and Immunogen D

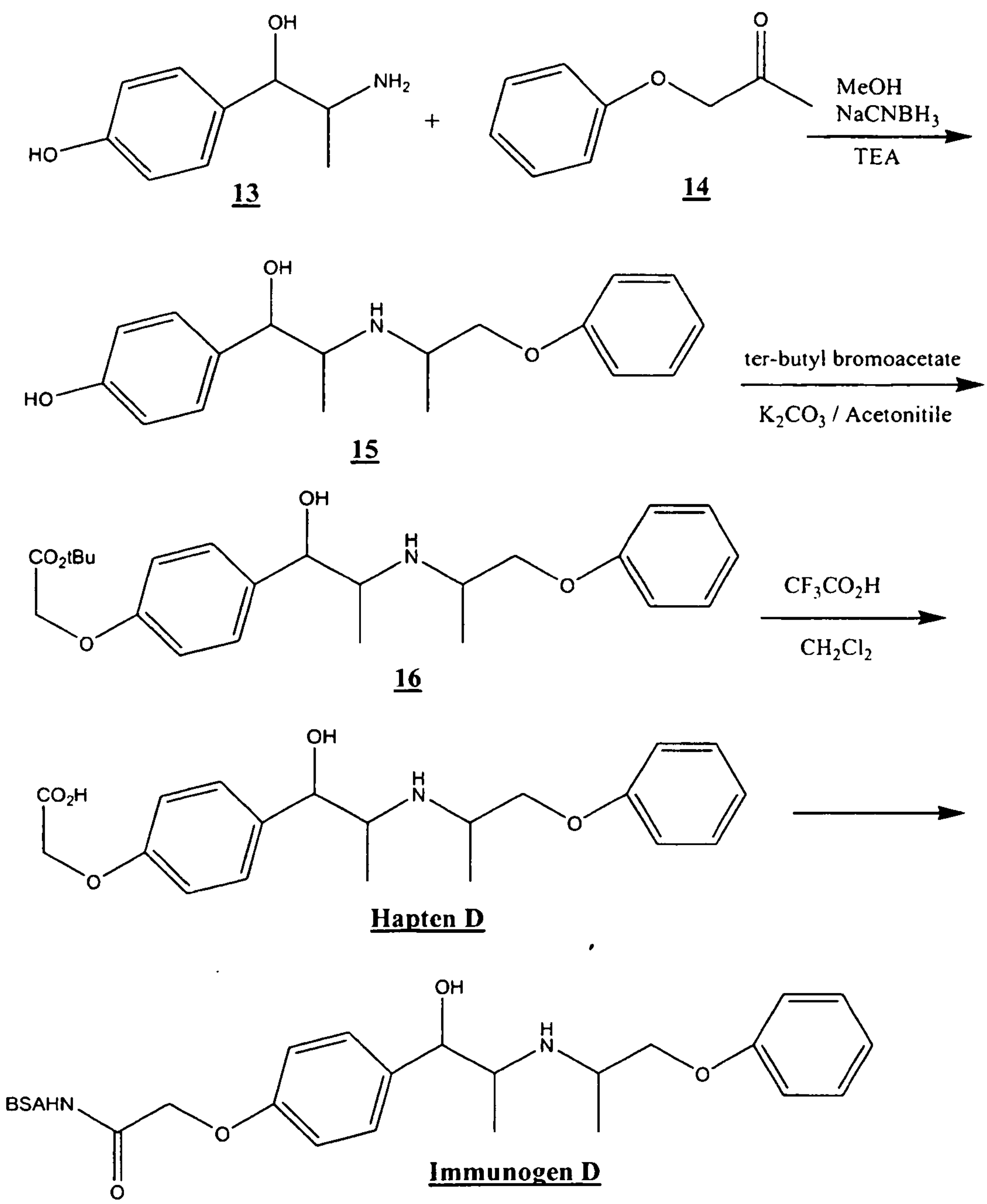


13

14

15

16

Hapten D

Immunogen D

**Figure - 6**

## Chemical reactions for the preparation of Hapten E and Immunogen E

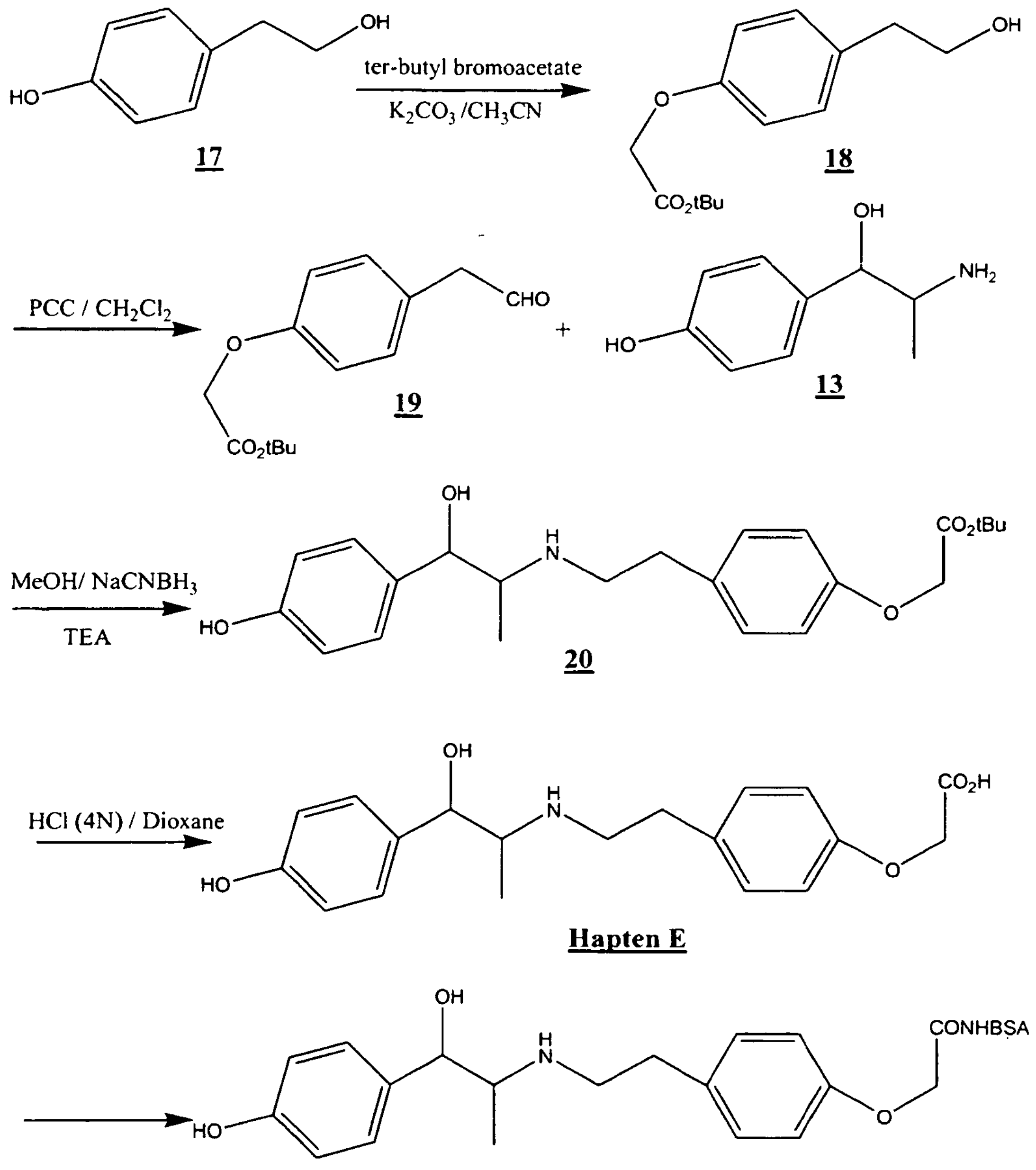


17

18

19

13

20

Hapten E

Immunogen E

**Figure - 7**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6274334 B
- US 6498010 A


### Non-patent literature cited in the description


- **Haasnoot et al.** Determination of fenoterol and ractopamine in urine by enzyme immunoassay. *The Analyst,* 1994, vol. 119, 2675-2680
- **Elliott et al.** Screening and confirmatory determination of ractopamine residues in calves treated with growth promoting doses of the β-agonist. *The Analyst,* 1998, vol. 123, 1103-1107
- **Shelver ; Smith.** Development of an immunoassay for the β-adrenergic agonist ractopamine. *Journal of Immunoassay,* 2000, vol. 21 (1), 1-23
- **Shelver et al.** Production and characterisation of a monoclonal antibody against the β-adrenergic agonist ractopamine. *J. Agric. Food Chem.,* 2000, vol. 48, 4020-4026
- **Shelver ; Smith.** *Journal of Immunoassay,* 2000, vol. 21 (1
- **Shelver et al.** *J. Agric. Food Chem.,* 2000, vol. 48